(19) **Europäisches Patentamt / European Patent Office / Office européen des brevets**

(11) **EP 3 485 902 A1**

(12) **EUROPEAN PATENT APPLICATION**

(43) Date of publication:
22.05.2019 Bulletin 2019/21

(21) Application number: 18189626.7

(22) Date of filing: 14.10.2014

(51) Int Cl.:
A61K 39/00 (2006.01)     A61K 39/395 (2006.01)
G01N 33/48 (2006.01)     A61P 11/00 (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 15.10.2013  US 201361891175 P
25.03.2014  US 201461970126 P

(62) Document number(s) of the earlier application(s) in accordance with Art. 76 EPC:
**14853201.3 / 3 057 609**

(71) Applicant: **Astrazeneca AB**
**151 85 Södertälje (SE)**

(72) Inventors:
• **VAN DER MERWE, Rene**
**Cambridge, Cambridgeshire CB21 6GH (GB)**

• **WARD, Christine**
**Gaithersburg, Maryland 20878 (US)**
• **MARTIN, Ubaldo**
**Gaithersburg, Maryland 20878 (US)**
• **ROSKOS, Lorin**
**Gaithersburg, Maryland 20878 (US)**
• **WANG, Bing**
**Gaithersburg, Maryland 20878 (US)**

(74) Representative: **AstraZeneca**
**Milstein Building**
**Granta Park**
**Cambridge CB21 6GH (GB)**

Remarks:
This application was filed on 17-08-2018 as a divisional application to the application mentioned under INID code 62.

(54) **METHODS FOR TREATING CHRONIC OBSTRUCTIVE PULMONARY DISEASE USING BENRALIZUMAB**

(57)     Provided herein is are methods of treating Chronic Obstructive Pulmonary Disease (COPD) in a patient, comprising administering to the patient an effective amount of benralizumab or an antigen-binding fragment thereof.

SEQUENCE LISTING

<110> MEDIMMUNE, LLC

<120> METHODS FOR TREATING CHRONIC OBSTRUCTIVE PULMONARY DISEASE USING
       BENRALIZUMAB

<130> IL5R-606WO1

<140>
<141>

<150> 61/970,126
<151> 2014-03-25

<150> 61/891,175
<151> 2013-10-15

<160> 12

<170> PatentIn version 3.5

<210> 1
<211> 107
<212> PRT
<213> Homo sapiens

<400> 1
Asp Ile Gln Met Thr Gln Ser Pro Ser Ser Leu Ser Ala Ser Val Gly
1               5                   10                  15


Asp Arg Val Thr Ile Thr Cys Gly Thr Ser Glu Asp Ile Ile Asn Tyr
            20                  25                  30


Leu Asn Trp Tyr Gln Gln Lys Pro Gly Lys Ala Pro Lys Leu Leu Ile
            35                  40                  45


Tyr His Thr Ser Arg Leu Gln Ser Gly Val Pro Ser Arg Phe Ser Gly
        50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Gly Tyr Thr Leu Pro Tyr
                85                  90                  95


Thr Phe Gly Gln Gly Thr Lys Val Glu Ile Lys
                100                 105


<210> 2
<211> 214
<212> PRT
<213> Homo sapiens

<400> 2
Asp Ile Gln Met Thr Gln Ser Pro Ser Ser Leu Ser Ala Ser Val Gly

```
        1                   5                   10                  15

        Asp Arg Val Thr Ile Thr Cys Gly Thr Ser Glu Asp Ile Ile Asn Tyr
                    20                  25                  30

        Leu Asn Trp Tyr Gln Gln Lys Pro Gly Lys Ala Pro Lys Leu Leu Ile
                    35                  40                  45

        Tyr His Thr Ser Arg Leu Gln Ser Gly Val Pro Ser Arg Phe Ser Gly
            50                  55                  60

        Ser Gly Ser Gly Thr Asp Phe Thr Leu Thr Ile Ser Ser Leu Gln Pro
        65                  70                  75                  80

        Glu Asp Phe Ala Thr Tyr Tyr Cys Gln Gln Gly Tyr Thr Leu Pro Tyr
                    85                  90                  95

        Thr Phe Gly Gln Gly Thr Lys Val Glu Ile Lys Arg Thr Val Ala Ala
                    100                 105                 110

        Pro Ser Val Phe Ile Phe Pro Pro Ser Asp Glu Gln Leu Lys Ser Gly
                    115                 120                 125

        Thr Ala Ser Val Val Cys Leu Leu Asn Asn Phe Tyr Pro Arg Glu Ala
                    130                 135                 140

        Lys Val Gln Trp Lys Val Asp Asn Ala Leu Gln Ser Gly Asn Ser Gln
        145                 150                 155                 160

        Glu Ser Val Thr Glu Gln Asp Ser Lys Asp Ser Thr Tyr Ser Leu Ser
                    165                 170                 175

        Ser Thr Leu Thr Leu Ser Lys Ala Asp Tyr Glu Lys His Lys Val Tyr
                    180                 185                 190

        Ala Cys Glu Val Thr His Gln Gly Leu Ser Ser Pro Val Thr Lys Ser
                    195                 200                 205

        Phe Asn Arg Gly Glu Cys
            210


<210> 3
<211> 121
<212> PRT
<213> Homo sapiens

<400> 3
Glu Val Gln Leu Val Gln Ser Gly Ala Glu Val Lys Lys Pro Gly Ala
1                   5                   10                  15
```

```
Ser Val Lys Val Ser Cys Lys Ala Ser Gly Tyr Thr Phe Thr Ser Tyr
            20                  25                  30

Val Ile His Trp Val Arg Gln Arg Pro Gly Gln Gly Leu Ala Trp Met
            35                  40                  45

Gly Tyr Ile Asn Pro Tyr Asn Asp Gly Thr Lys Tyr Asn Glu Arg Phe
            50                  55                  60

Lys Gly Lys Val Thr Ile Thr Ser Asp Arg Ser Thr Ser Thr Val Tyr
65                      70                  75                  80

Met Glu Leu Ser Ser Leu Arg Ser Glu Asp Thr Ala Val Tyr Leu Cys
                85                  90                  95

Gly Arg Glu Gly Ile Arg Tyr Tyr Gly Leu Leu Gly Asp Tyr Trp Gly
            100                 105                 110

Gln Gly Thr Leu Val Thr Val Ser Ser
            115                 120


<210> 4
<211> 451
<212> PRT
<213> Homo sapiens

<400> 4
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Val Ile His Trp Val Arg Gln Arg Pro Gly Gln Gly Leu Ala Trp Met
            35                  40                  45

Gly Tyr Ile Asn Pro Tyr Asn Asp Gly Thr Lys Tyr Asn Glu Arg Phe
            50                  55                  60

Lys Gly Lys Val Thr Ile Thr Ser Asp Arg Ser Thr Ser Thr Val Tyr
65                      70                  75                  80

Met Glu Leu Ser Ser Leu Arg Ser Glu Asp Thr Ala Val Tyr Leu Cys
                85                  90                  95

Gly Arg Glu Gly Ile Arg Tyr Tyr Gly Leu Leu Gly Asp Tyr Trp Gly
            100                 105                 110
```

```
Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
        130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
                180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
        210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
                260                 265                 270

Glu Asp Pro Glu Val Lys Phe Asn Trp Tyr Val Asp Gly Val Glu Val
                275                 280                 285

His Asn Ala Lys Thr Lys Pro Arg Glu Glu Gln Tyr Asn Ser Thr Tyr
        290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
        340                 345                 350

Tyr Thr Leu Pro Pro Ser Arg Asp Glu Leu Thr Lys Asn Gln Val Ser
        355                 360                 365
```

```
Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
            435                 440                 445

Pro Gly Lys
        450


<210> 5
<211> 400
<212> PRT
<213> Homo sapiens

<400> 5
Asp Leu Leu Pro Asp Glu Lys Ile Ser Leu Leu Pro Pro Val Asn Phe
1               5               10                  15

Thr Ile Lys Val Thr Gly Leu Ala Gln Val Leu Leu Gln Trp Lys Pro
            20                  25                  30

Asn Pro Asp Gln Glu Gln Arg Asn Val Asn Leu Glu Tyr Gln Val Lys
            35                  40                  45

Ile Asn Ala Pro Lys Glu Asp Asp Tyr Glu Thr Arg Ile Thr Glu Ser
    50                  55                  60

Lys Cys Val Thr Ile Leu His Lys Gly Phe Ser Ala Ser Val Arg Thr
65                  70                  75                  80

Ile Leu Gln Asn Asp His Ser Leu Leu Ala Ser Ser Trp Ala Ser Ala
                85                  90                  95

Glu Leu His Ala Pro Pro Gly Ser Pro Gly Thr Ser Ile Val Asn Leu
            100                 105                 110

Thr Cys Thr Thr Asn Thr Thr Glu Asp Asn Tyr Ser Arg Leu Arg Ser
        115                 120                 125
```

```
Tyr Gln Val Ser Leu His Cys Thr Trp Leu Val Gly Thr Asp Ala Pro
    130                 135                 140

Glu Asp Thr Gln Tyr Phe Leu Tyr Tyr Arg Tyr Gly Ser Trp Thr Glu
    145                 150                 155                 160

Glu Cys Gln Glu Tyr Ser Lys Asp Thr Leu Gly Arg Asn Ile Ala Cys
                165                 170                 175

Trp Phe Pro Arg Thr Phe Ile Leu Ser Lys Gly Arg Asp Trp Leu Ala
            180                 185                 190

Val Leu Val Asn Gly Ser Ser Lys His Ser Ala Ile Arg Pro Phe Asp
        195                 200                 205

Gln Leu Phe Ala Leu His Ala Ile Asp Gln Ile Asn Pro Pro Leu Asn
    210                 215                 220

Val Thr Ala Glu Ile Glu Gly Thr Arg Leu Ser Ile Gln Trp Glu Lys
225                 230                 235                 240

Pro Val Ser Ala Phe Pro Ile His Cys Phe Asp Tyr Glu Val Lys Ile
            245                 250                 255

His Asn Thr Arg Asn Gly Tyr Leu Gln Ile Glu Lys Leu Met Thr Asn
            260                 265                 270

Ala Phe Ile Ser Ile Ile Asp Asp Leu Ser Lys Tyr Asp Val Gln Val
        275                 280                 285

Arg Ala Ala Val Ser Ser Met Cys Arg Glu Ala Gly Leu Trp Ser Glu
    290                 295                 300

Trp Ser Gln Pro Ile Tyr Val Gly Asn Asp Glu His Lys Pro Leu Arg
305                 310                 315                 320

Glu Trp Phe Val Ile Val Ile Met Ala Thr Ile Cys Phe Ile Leu Leu
                325                 330                 335

Ile Leu Ser Leu Ile Cys Lys Ile Cys His Leu Trp Ile Lys Leu Phe
            340                 345                 350

Pro Pro Ile Pro Ala Pro Lys Ser Asn Ile Lys Asp Leu Phe Val Thr
            355                 360                 365

Thr Asn Tyr Glu Lys Ala Gly Ser Ser Glu Thr Glu Ile Glu Val Ile
    370                 375                 380
```

```
Cys Tyr Ile Glu Lys Pro Gly Val Glu Thr Leu Glu Asp Ser Val Phe
385             390             395                 400
```

<210> 6
<211> 398
<212> PRT
<213> Mus musculus

<400> 6

```
Asp Leu Leu Asn His Lys Lys Phe Leu Leu Leu Pro Pro Val Asn Phe
1               5               10              15


Thr Ile Lys Ala Thr Gly Leu Ala Gln Val Leu Leu His Trp Asp Pro
            20              25              30


Asn Pro Asp Gln Glu Gln Arg His Val Asp Leu Glu Tyr His Val Lys
            35              40              45


Ile Asn Ala Pro Gln Glu Asp Glu Tyr Asp Thr Arg Lys Thr Glu Ser
    50              55              60


Lys Cys Val Thr Pro Leu His Glu Gly Phe Ala Ala Ser Val Arg Thr
65              70              75              80


Ile Leu Lys Ser Ser His Thr Thr Leu Ala Ser Ser Trp Val Ser Ala
                85              90              95


Glu Leu Lys Ala Pro Pro Gly Ser Pro Gly Thr Ser Val Thr Asn Leu
            100             105             110


Thr Cys Thr Thr His Thr Val Val Ser Ser His Thr His Leu Arg Pro
            115             120             125


Tyr Gln Val Ser Leu Arg Cys Thr Trp Leu Val Gly Lys Asp Ala Pro
    130             135             140


Glu Asp Thr Gln Tyr Phe Leu Tyr Tyr Arg Phe Gly Val Leu Thr Glu
145             150             155             160


Lys Cys Gln Glu Tyr Ser Arg Asp Ala Leu Asn Arg Asn Thr Ala Cys
            165             170             175


Trp Phe Pro Arg Thr Phe Ile Asn Ser Lys Gly Phe Glu Gln Leu Ala
            180             185             190


Val His Ile Asn Gly Ser Ser Lys Arg Ala Ala Ile Lys Pro Phe Asp
            195             200             205
```

```
Gln Leu Phe Ser Pro Leu Ala Ile Asp Gln Val Asn Pro Pro Arg Asn
    210                 215                 220

Val Thr Val Glu Ile Glu Ser Asn Ser Leu Tyr Ile Gln Trp Glu Lys
225                 230                 235                 240

Pro Leu Ser Ala Phe Pro Asp His Cys Phe Asn Tyr Glu Leu Lys Ile
                245                 250                 255

Tyr Asn Thr Lys Asn Gly His Ile Gln Lys Glu Lys Leu Ile Ala Asn
                260                 265                 270

Lys Phe Ile Ser Lys Ile Asp Asp Val Ser Thr Tyr Ser Ile Gln Val
                275                 280                 285

Arg Ala Ala Val Ser Ser Pro Cys Arg Met Pro Gly Arg Trp Gly Glu
    290                 295                 300

Trp Ser Gln Pro Ile Tyr Val Gly Lys Glu Arg Lys Ser Leu Val Glu
305                 310                 315                 320

Trp His Leu Ile Val Leu Pro Thr Ala Ala Cys Phe Val Leu Leu Ile
                325                 330                 335

Phe Ser Leu Ile Cys Arg Val Cys His Leu Trp Thr Arg Leu Phe Pro
                340                 345                 350

Pro Val Pro Ala Pro Lys Ser Asn Ile Lys Asp Leu Pro Val Val Thr
                355                 360                 365

Glu Tyr Glu Lys Pro Ser Asn Glu Thr Lys Ile Glu Val Val His Cys
    370                 375                 380

Val Glu Glu Val Gly Phe Glu Val Met Gly Asn Ser Thr Phe
385                 390                 395
```

```
<210> 7
<211> 5
<212> PRT
<213> Homo sapiens

<400> 7
Ser Tyr Val Ile His
1               5


<210> 8
<211> 17
<212> PRT
<213> Homo sapiens
```

```
<400> 8
Tyr Ile Asn Pro Tyr Asn Asp Gly Thr Lys Tyr Asn Glu Arg Phe Lys
1               5               10                  15


Gly



<210> 9
<211> 12
<212> PRT
<213> Homo sapiens

<400> 9
Glu Gly Ile Arg Tyr Tyr Gly Leu Leu Gly Asp Tyr
1               5               10



<210> 10
<211> 11
<212> PRT
<213> Homo sapiens

<400> 10
Gly Thr Ser Glu Asp Ile Ile Asn Tyr Leu Asn
1               5               10



<210> 11
<211> 7
<212> PRT
<213> Homo sapiens

<400> 11
His Thr Ser Arg Leu Gln Ser
1               5



<210> 12
<211> 9
<212> PRT
<213> Homo sapiens

<400> 12
Gln Gln Gly Tyr Thr Leu Pro Tyr Thr
1               5
```

**Description**

BACKGROUND

Chronic obstructive pulmonary disease (COPD) is a significant cause of morbidity and mortality worldwide. In contrast to other chronic diseases, COPD is increasing in prevalence and is projected to be the third-leading cause of death and disability worldwide by 2020. The costs to society for treating COPD are high, accounting for approximately 3.4% of the total health care budget of the European Union. In the United States, the direct and indirect costs of COPD are estimated to be more than $30 billion.

Approximately 30% of patients with COPD have elevated levels of eosinophils in the airway as measured by sputum induction or bronchoalveolar lavage. In COPD, the response to oral and inhaled corticosteroids (ICS) is related to the intensity of the airway eosinophilic inflammation, and a sputum eosinophilia count of greater than 3% has been demonstrated to be a good predictor of response to steroids in COPD. A strategy in which increasing therapy with corticosteroids was used to control sputum eosinophilia greater than 3% in COPD resulted in a reduction in the frequency of severe COPD exacerbations requiring admission to a hospital when patients were stepped up to oral corticosteroid therapy. Standard therapy for acute exacerbations of COPD (AECOPD) includes treatment of inflammation with systemic corticosteroids, which are associated with a reduction in length of hospital stay and hastened recovery. Corticosteroids are responsible for early apoptosis of eosinophils and generally result in a reduction in eosinophilia. Unfortunately, long-term therapy with corticosteroids is associated with significant side effects such as suppression of the hypothalamic-pituitary-adrenal axis and osteoporosis, and corticosteroids do not avert exacerbations in all eosinophilic COPD patients.

COPD patients with increased sputum eosinophil counts have been shown to have significant improvements in forced expiratory volume in 1 second ($FEV_1$) and quality of life-scores that were associated with decreased sputum eosinophil counts and eosinophil cationic protein (ECP) levels. Thus, therapies specifically targeted at eosinophils in COPD may have beneficial effects.

Benralizumab is a humanized, afucosylated monoclonal antibody (mAb) that specifically binds to the alpha chain of human interleukin-5 receptor alpha (IL-5R$\alpha$), which is expressed on eosinophils. It induces apoptosis of these cells via antibody-dependent cell cytotoxicity.

Thus, given the high unmet need of treating COPD without the corticosteroid-induced side effects and the fact that some patients with COPD have an eosinophilic component, the effect of benralizumab on COPD in adult subjects was examined.

BRIEF SUMMARY

Methods of treating chronic obstructive pulmonary disease (COPD) in a human COPD patient are provided herein.

In certain aspects, a method of treating COPD comprises administering to a COPD patient a dose of 100 mg of benralizumab or an antigen-binding fragment thereof. In certain aspects, a method of reducing the exacerbation rate of COPD comprises administering to a human COPD patient an effective amount of benralizumab or an antigen-binding fragment thereof, wherein the patient has a blood eosinophil count of at least 200 eosinophils/$\mu$L prior to the administration. In certain aspects, a method of reducing the exacerbation rate of COPD comprises administering to a human COPD patient an effective amount of benralizumab or an antigen-binding fragment thereof, wherein the patient has severe or very severe COPD as defined by the Global Initiative for Chronic Obstructive Lung Disease (GOLD). In certain aspects, a method of increasing forced expiratory volume in one second ($FEV_1$) in a human COPD patient comprises administering an effective amount of benralizumab or an antigen-binding fragment thereof to the patient. In certain aspects, a method of increasing forced vital capacity (FVC) in a human COPD patient comprises administering an effective amount of benralizumab or an antigen-binding fragment thereof to the patient. In certain aspects, a method of improving a COPD questionnaire score assessing COPD symptoms in a human COPD patient comprises administering an effective amount of benralizumab or an antigen-binding fragment thereof to the patient.

In certain aspects, the benralizumab or antigen-binding fragment is administered in a dose of 100 mg. In certain aspects, the benralizumab or antigen-binding fragment is administered in a dose of 30 mg. In certain aspects, the benralizumab or antigen-binding fragment is administered in a dose of 10 mg.

In certain aspects, the patient has a blood eosinophil count of at least 200 eosinophils/$\mu$L prior to the administration. In certain aspects, the patient has a blood eosinophil count of at least 300 eosinophils/$\mu$L prior to the administration. In certain aspects, the patient has a blood eosinophil count of at least 400 eosinophils/$\mu$L prior to the administration.

In certain aspects, the patient has a blood eosinophil count of less than 150 eosinophils/$\mu$L prior to the administration. In certain aspects, the patient has a blood eosinophil count of less than 300 eosinophils/$\mu$L prior to the administration. In certain aspects, the patient has a blood eosinophil count of 150-300 eosinophils/$\mu$L prior to the administration.

In certain aspects, the patient has a blood eosinophil count of 300-450 eosinophils/$\mu$L prior to the administration. In certain aspects, the patient has a blood eosinophil count of greater than 400 eosinophils/$\mu$L prior to the administration. In certain aspects, the patient has a blood eosinophil count of greater than 450 eosinophils/$\mu$L prior to the administration.

In certain aspects, the patient has severe or very severe COPD as defined by GOLD. In certain aspects, the patient has very severe COPD as defined by GOLD.

In certain aspects, the administration reduces the exacerbation rate of COPD. In certain aspects, the exacerbation rate is reduced by at least 30%. In certain aspects, the exacerbation rate is reduced by about 34%. In certain aspects, the exacerbation rate is reduced by at least 40%. In certain aspects, the exacerbation rate is reduced by about 47%. In certain aspects, the exacerbation rate is reduced by at least 50%. In certain aspects, the exacerbation rate is reduced by about 57%. In certain aspects, the exacerbation rate is reduced within a year from the first administration of the benralizumab or antigen-binding fragment thereof.

In certain aspects, the administration increases the patient's $FEV_1$. In certain aspects, the increased $FEV_1$ is a pre-bronchodilator $FEV_1$. In certain aspects, the pre-bronchodilator $FEV_1$ is increased by at least 10%. In certain aspects, the pre-bronchodilator $FEV_1$ is increased by about 12%. In certain aspects, the increased $FEV_1$ is a post-bronchodilator $FEV_1$. In certain aspects, the post-bronchodilator $FEV_1$ is increased by at least 5%. In certain aspects, the post-bronchodilator $FEV_1$ is increased by about 7%. In certain aspects, the pre-bronchodilator $FEV_1$ and the post-bronchodilator $FEV_1$ increase. In certain aspects, the $FEV_1$ is increased within a year from the first administration of the benralizumab or antigen-binding fragment thereof.

In certain aspects, the administration increases the patient's FVC. In certain aspects, the increased FVC is a pre-bronchodilator FVC. In certain aspects, the increased FVC is a post-bronchodilator FVC. In certain aspects, the pre-bronchodilator FVC and the post-bronchodilator FVC increase. In certain aspects, the FVC is increased by at least 3%. In certain aspects, the FVC is increased within a year from the first administration of the benralizumab or antigen-binding fragment thereof.

In certain aspects, the administration improves a COPD questionnaire score assessing COPD symptoms. In certain aspects, the COPD questionnaire is the COPD-Specific Saint George's Respiratory Questionnaire (SGRQ-C). In certain aspects, the patient's SGRQ-C (symptom) score decreases by at least 9. In certain aspects, the COPD questionnaire score assessing COPD symptoms improve with a year from the first administration of the benralizumab or antigen-binding fragment thereof.

In certain aspects, the patient has a history of exacerbations. In certain aspects, the history of exacerbations comprises at least one exacerbation in the year prior to the administration of the benralizumab or antigen-binding fragment thereof.

In certain aspects, the patient had an $FEV_1$ < 80% predicted prior to the administration of the benralizumab or antigen-binding fragment thereof.

In certain aspects, the patient had an $FEV_1$/ forced vital capacity (FVC) < 0.70 prior to the administration of the benralizumab or antigen-binding fragment thereof.

In certain aspects, the patient uses corticosteroids, long-acting $\beta2$ agonists, and tiotropium.

In certain aspects, at least two doses of the benralizumab or antigen-binding fragment thereof are administered. In certain aspects, a first dose of benralizumab or antigen-binding fragment thereof is administered at day zero and a second dose is administered at 4 weeks. In certain aspects, at least one dose of the benralizumab or antigen-binding fragment thereof is administered at an interval of 8 weeks after the previous dose. In certain aspects, the benralizumab or antigen-binding fragment thereof is administered with at least one four-week dosing interval and then with at least one eight-week dosing interval. In certain aspects, the benralizumab or antigen-binding fragment thereof is administered with three four-week dosing intervals and then at eight-week dosing intervals.

In certain aspects, the administration is subcutaneous.

In certain aspects, a method of treating COPD in a human COPD patient, comprises administering to the patient a dose of 100 mg of benralizumab or an antigen-binding fragment thereof, wherein the patient has a blood eosinophil count of at least 200 eosinophils/$\mu$L prior to the administration. In certain aspects, the patient has a blood eosinophil count of at least 300 eosinophils/$\mu$L prior to the administration. In certain aspects, the patient has a blood eosinophil count of at least 400 eosinophils/$\mu$L prior to the administration. In certain aspects, the benralizumab or antigen-binding fragment thereof is administered with at least one four-week dosing interval and then with at least one eight-week dosing interval. In certain aspects, the administration of the benralizumab or antigen-binding fragment thereof decreases the exacerbation rate of chronic obstructive pulmonary disease (COPD), increases the patient's $FEV_1$, improves a COPD questionnaire score assessing COPD symptoms, or a combination thereof. In certain aspects, the administration of the benralizumab or antigen-binding fragment thereof decreases the exacerbation rate of chronic obstructive pulmonary disease (COPD), increases the patient's $FEV_1$, and improves a COPD questionnaire score assessing COPD symptoms.

In certain aspects, a method of reducing the exacerbation rate of COPD comprises administering to a human COPD patient an effective amount of benralizumab or an antigen-binding fragment thereof, wherein the patient has a blood eosinophil count of at least 200 eosinophils/$\mu$L prior to the administration and wherein the exacerbation rate is reduced by at least 30%. In certain aspects, the exacerbation rate is reduced by about 34%.

In certain aspects, a method of reducing the exacerbation rate of COPD comprises administering to a human COPD patient an effective amount of benralizumab or an antigen-binding fragment thereof, wherein the patient has a blood eosinophil count of at least 300 eosinophils/$\mu$L prior to the administration and wherein the exacerbation rate is reduced by at least 50%. In certain aspects, the exacerbation rate is reduced by about 57%

In certain aspects, a method of reducing the exacerbation rate of COPD comprises administering to a human COPD patient an effective amount of benralizumab or an antigen-binding fragment thereof, wherein the patient has severe or very severe COPD as defined by GOLD and wherein the exacerbation rate is reduced by at least 40%. In certain aspects, the exacerbation rate is reduced by about 47%

In certain aspects, a method of increasing $FEV_1$ in a human COPD patient comprises administering an effective amount of benralizumab or an antigen-binding fragment thereof to the patient, wherein the patient has a blood eosinophil count of at least 200 eosinophils/$\mu$L prior to the administration. In certain aspects, a method of increasing $FEV_1$ in a human COPD patient comprises administering an effective amount of benralizumab or an antigen-binding fragment thereof to the patient, wherein the patient uses corticosteroids, long-acting $\beta$2 agonists, and tiotropium. In certain aspects, the pre-bronchodilator $FEV_1$ increases. In certain aspects, the pre-bronchodilator $FEV_1$ increases by at least 15%. In certain aspects, the post-bronchodilator $FEV_1$ increases. In certain aspects, the post-bronchodilator $FEV_1$ increases by at least 10%. In certain aspects, the pre-bronchodilator $FEV_1$ increases and the post-bronchodilator $FEV_1$ increases. In certain aspects, the pre-bronchodilator $FEV_1$ increases by at least 15% and the post-bronchodilator $FEV_1$ increases by at least 10%.

In certain aspects, a method of increasing $FEV_1$ in a human COPD patient comprises administering an effective amount of benralizumab or an antigen-binding fragment thereof to the patient, wherein the patient has severe or very severe COPD as defined by GOLD. In certain aspects, the pre-bronchodilator $FEV_1$ increases. In certain aspects, the pre-bronchodilator $FEV_1$ increases by at least 20%. In certain aspects, the post-bronchodilator $FEV_1$ increases. In certain aspects, the post-bronchodilator $FEV_1$ increases by at least 15%. In certain aspects, the pre-bronchodilator $FEV_1$ increases and the post-bronchodilator $FEV_1$ increases. In certain aspects, the pre-bronchodilator $FEV_1$ increases by at least 20% and the post-bronchodilator $FEV_1$ increases by at least 15%.

In certain aspects, the administration of benralizumab or an antigen-binding fragment thereof reduces the annual COPD exacerbation rate.

In certain aspects, the administration of benralizumab or an antigen-binding fragment thereof improves a Specific Saint George's Respiratory Questionnaire (SGRQ) score.

In certain aspects, a method of treating COPD comprises administering to a COPD patient a dose of 30 mg of benralizumab or an antigen-binding fragment thereof. In certain aspects, a method of treating COPD comprises administering to a COPD patient a dose of 10 mg of benralizumab or an antigen-binding fragment thereof. In certain aspects, the administration reduces the annual COPD exacerbation rate.

In certain aspects, a method of reducing the annual exacerbation rate of COPD, comprises administering to a human COPD patient an effective amount of benralizumab or an antigen-binding fragment thereof.

In certain aspects, a COPD patient has a blood eosinophil count of at least 200 eosinophils/$\mu$L prior to the administration. In certain aspects, a COPD patient has a blood eosinophil count of at least 300 eosinophils/$\mu$L prior to the administration. In certain aspects, a COPD patient has a blood eosinophil count of less than 150 eosinophils/$\mu$L prior to the administration. In certain aspects, a COPD patient has a blood eosinophil count of less than 300 eosinophils/$\mu$L prior to the administration. In certain aspects, a COPD patient has a blood eosinophil count of 150-300 eosinophils/$\mu$L prior to the administration. In certain aspects, a COPD patient has a blood eosinophil count of 300-450 eosinophils/$\mu$L prior to the administration. In certain aspects, a COPD patient has a blood eosinophil count of at least 400 eosinophils/$\mu$L prior to the administration. In certain aspects, a COPD patient has a blood eosinophil count of at least 450 eosinophils/$\mu$L prior to the administration.

In certain aspects, a COPD patient uses an inhaled corticosteroids (ICS) and a long-acting beta agonist (LABA). In certain aspects, a COPD patient uses a LABA and long-acting muscarinic antagonist (LAMA). In certain aspects, a COPD patient uses ICS/LABA/LAMA.

In certain aspects, a COPD patient has an $FEV_1 < 50\%$ of the predicted normal value prior to the administration. In certain aspects, a COPD patient has a history of at least 1 COPD exacerbation in the year prior to the administration. In certain aspects, a COPD patient has severe or very severe COPD as defined by GOLD.

In certain aspects, at least two doses of the benralizumab or antigen-binding fragment thereof are administered. In certain aspects, the first dose of benralizumab or antigen-binding fragment thereof is administered at day zero and the second dose is administered at 4 weeks. In certain aspects, at least one dose of the benralizumab or antigen-binding fragment thereof is administered at an interval of 8 weeks after the previous dose. In certain aspects, the benralizumab or antigen-binding fragment thereof is administered with at least one four-week dosing interval and then with at least one eight-week dosing interval. In certain aspects, the benralizumab or antigen-binding fragment thereof is administered with three four-week dosing intervals and then at eight-week dosing intervals.

In certain aspects, the administration is subcutaneous.

In certain aspects of the provided methods, administration of the antibody or antigen-binding fragment thereof result in treatment of COPD as shown in Examples 1-4.

BRIEF DESCRIPTION OF THE DRAWINGS/FIGURES

**Figure 1** shows the study flow diagram described in Examples 1 and 2.

**Figure 2** shows the COPD exacerbation rate reduction in the Intent-to-Treat (ITT) population and various subgroups.

**Figure 3** shows the change from baseline in pre-bronchodilator $FEV_1$ predicted over time in the Per Protocol Population (PPP).

**Figure 4** shows the change from baseline in pre-bronchodilator $FEV_1$ (L) in the overall ITT population and various subgroups at day 393.

**Figure 5** shows the change from baseline in post-bronchodilator $FEV_1$ (L) in the overall ITT population and various subgroups at day 393.

**Figure 6** shows the change from baseline in COPD-Specific Saint George's Respiratory Questionnaire (SGRQ-C) total score in the overall ITT population and various subgroups.

**Figure 7** shows the change from baseline in SGRQ-C symptom score in the overall ITT population and various subgroups.

**Figure 8** shows the peripheral eosinophil count over time in the safety population.

**Figure 9** shows the sputum eosinophil count over time in the safety population.

**Figure 10** shows the basophil count over time in the safety population.

**Figure 11** shows the two-dose study flow diagram described in Example 3.

**Figure 12** shows the three-dose study flow diagram described in Example 3.

DETAILED DESCRIPTION

It is to be noted that the term "a" or "an" entity refers to one or more of that entity; for example, "an anti-IL-5$\alpha$ antibody" is understood to represent one or more anti-IL-5$\alpha$ antibodies. As such, the terms "a" (or "an"), "one or more," and "at least one" can be used interchangeably herein.

Provided herein are methods for treating Chronic Obstructive Pulmonary Disease (COPD). The methods provided include administering an effective amount of benralizumab or an antigen-binding fragment thereof.

Information regarding benralizumab (or fragments thereof) for use in the methods provided herein can be found, *e.g.*, in U.S. Patent Application Publication No. US 2010/0291073 A1, the disclosure of which is incorporated herein by reference in its entirety. Benralizumab and antigen-binding fragments thereof for use in the methods provided herein comprise a heavy chain and a light chain or a heavy chain variable region and a light chain variable region. In a further aspect, benralizumab or an antigen-binding fragment thereof for use in the methods provided herein includes any one of the amino acid sequences of SEQ ID NOs: 1-4. In a specific aspect, benralizumab or an antigen-binding fragment thereof for use in the methods provided herein comprises a light chain variable region comprising the amino acid sequence of SEQ ID NO:1 and a heavy chain variable region comprising the amino acid sequence of SEQ ID NO:3. In a specific aspect, benralizumab or an antigen-binding fragment thereof for use in the methods provided herein comprises a light chain comprising the amino acid sequence of SEQ ID NO: 2 and heavy chain comprising the amino acid sequence of SEQ ID NO:4. In a specific aspect, benralizumab or an antigen-binding fragment thereof for use in the methods provided herein comprises a heavy chain variable region and a light chain variable region, wherein the heavy chain variable region comprises the Kabat-defined CDR1, CDR2, and CDR3 sequences of SEQ ID NOs: 7-9, and wherein the light chain variable region comprises the Kabat-defined CDR1, CDR2, and CDR3 sequences of SEQ ID NOs: 10-12. Those of ordinary skill in the art would easily be able to identify Chothia-defined, Abm-defined or other CDRs. In a specific aspect, benralizumab or an antigen-binding fragment thereof for use in the methods provided herein comprises the variable heavy chain and variable light chain CDR sequences of the KM1259 antibody as disclosed in U.S. 6,018,032, which is herein incorporated by reference in its entirety.

An acute exacerbation of COPD (AECOPD) is a sustained worsening of a patient's condition from the stable state and beyond normal day-to-day variations that is acute in onset and necessitates a change in regular medication in a patient with underlying COPD.

In certain aspects, a patient presenting at a physician's office or emergency department (ED) with COPD is administered benralizumab or an antigen-binding fragment thereof. Given the ability of benralizumab to reduce or deplete eosinophil counts for up to 12 weeks or more (*see* US 2010/0291073), benralizumab or an antigen-binding fragment thereof can be administered only once or infrequently while still providing benefit to the patient. In further aspects, the patient is administered additional follow-on doses. Follow-on doses can be administered at various time intervals depending on the patient's age, weight, ability to comply with physician instructions, clinical assessment, eosinophil count (blood or sputum eosinophils or eosinophilic cationic protein (ECP) measurement), or and other factors, including the judgment of the attending physician. The intervals between doses can be every 4 weeks, every 5 weeks, every 6 weeks, every 8 weeks, every 10 weeks, every 12 weeks, or longer intervals. In certain aspects, the intervals between doses can be every 4 weeks or every 8 weeks. In certain aspects, the intervals between doses can be every 4 weeks and every 8 weeks. In certain aspects, benralizumab or an antigen-binding fragment thereof is administered with three four-week dosing intervals (i.e., on Day 0, Week 4, and Week 8) and then with eight-week dosing intervals (i.e., on Week 16, Week 24, Week 32, etc.).

In certain aspects, the single dose or first dose is administered to the COPD patient shortly after the patient presents with an acute exacerbation, e.g., a mild, moderate or severe exacerbation. For example, the single or first dose of benralizumab or an antigen-binding fragment thereof can be administered during the presenting clinic or hospital visit, or in the case of very severe exacerbations, within 1, 2, 3, 4, 5, 6, 7, or more days, *e.g.*, 7 days of the acute exacerbation, allowing the patient's symptoms to stabilize prior to administration of benralizumab.

In some embodiments, at least two doses of benralizumab or antigen-binding fragment thereof are administered to the patient. In some embodiments, at least three doses, at least four doses, at least five doses, at least six doses, or at least seven doses are administered to the patient. In some embodiments, benralizumab or an antigen-binding fragment thereof is administered over the course of four weeks, over the course of eight weeks, over the course of twelve weeks, over the course of twenty-four weeks, over the course of forty-eight weeks, or over the course of a year or more.

The amount of benralizumab or an antigen-binding fragment thereof to be administered to the patient can depend on various parameters such as the patient's age, weight, clinical assessment, eosinophil count (blood or sputum eosinophils, eosinophilic cationic protein (ECP) measurement, or eosinophil derived neurotoxin (EDN) measurement), or and other factors, including the judgment of the attending physician. In certain aspects, the dosage or dosage interval is not dependent on the eosinophil level.

In certain aspects, the patient is administered one or more doses of benralizumab or an antigen-binding fragment thereof wherein the dose is about 100 mg. In certain aspects, the patient is administered one or more doses of benralizumab or an antigen-binding fragment thereof wherein the dose is about 30 mg. In certain aspects, the patient is administered one or more doses of benralizumab or an antigen-binding fragment thereof wherein the dose is about 10 mg.

In certain aspects, administration of benralizumab or an antigen-binding fragment thereof according to the methods provided herein is through parenteral administration. For example, benralizumab or an antigen-binding fragment thereof can be administered by intravenous infusion or by subcutaneous injection. In certain embodiments, benralizumab or an antigen-binding fragment thereof can be administered by subcutaneous injection.

In certain aspects, benralizumab or an antigen-binding fragment thereof is administered according to the methods provided herein in combination or in conjunction with additional therapies. Such therapies include, without limitation, corticosteroid therapy (including inhaled corticosteroids (ICS)), long-acting $\beta$ agonists (LABA, including long-acting $\beta2$ agonists), tiotropium, or other standard therapies. In certain aspects, benralizumab or an antigen-binding fragment there of is administered according to the methods provided herein in combination or in conjunction with ICS and LABA, with LABA and LAMA, or with ICS, LABA, and LAMA.

In certain instances, administration of benralizumab or an antigen-binding fragment thereof decreases COPD exacerbations including, for example, as measured by an exacerbation rate, an annual exacerbation rate, time to first exacerbation, and/or an annual rate of COPD exacerbations that are associated with an emergency room visit or hospitalization.

The methods provided herein can reduce exacerbation rates in COPD patients. In certain aspects, use of the methods provided herein, *i.e.*, administration of benralizumab or an antigen-binding fragment thereof reduces the number of exacerbations experienced by the patient as compared to the number of exacerbations expected according to the patient's history, as compared to the average number of exacerbations expected in a comparable population of patients, or as compared to a comparable population treated with placebo over the same time period. In certain aspects, administration of benralizumab or an antigen-binding fragment thereof reduces the number of exacerbations in COPD patients with eosinophil counts of at least 200 eosinophils/$\mu$L prior to the administration. In certain aspects, administration of benralizumab or an antigen-binding fragment thereof reduces the number of exacerbations in COPD patients with eosinophil counts of at least 300 eosinophils/$\mu$L prior to the administration. In certain aspects, administration of benralizumab or an antigen-binding fragment thereof reduces the number of exacerbations in COPD patients with eosinophil counts of at least 400 eosinophils/$\mu$L prior to the administration. In certain aspects, administration of benralizumab or an antigen-binding fragment thereof reduces the number of exacerbations in COPD patients with severe COPD as defined by the Global Initiative for Chronic Obstructive Lung Disease (GOLD), Global strategy for the diagnosis, management, and prevention of chronic obstructive pulmonary disease (updated 2009). In certain aspects, administration of benralizumab or an antigen-binding fragment thereof reduces the number of exacerbations in COPD patients with very severe COPD as defined by the GOLD. In certain aspects, administration of benralizumab or an antigen-binding fragment thereof reduces the number of exacerbations in COPD patients with severe or very severe COPD as defined by the GOLD. In certain aspects, administration of benralizumab or an antigen-binding fragment thereof reduces the number of exacerbations in COPD patients who are receiving corticosteroids (e.g., inhaled corticosteroids (ICS), long-acting β-agonists (LABA) (e.g., long-acting β2-agonists), and tiotropium.

In certain aspects, administration of benralizumab or an antigen-binding fragment thereof reduces exacerbations by at least about 15%, by at least about 20%, by at least about 25%, by at least about 30%, at least about 35%, at least about 40%, at least about 45%, at least about 50%, or at least about 55%. In some embodiments, exacerbations are reduced about 34%, about 47%, or about 57%. The exacerbations can be reduced, for example, within a year from the first administration of benralizumab or the antigen-binding fragment thereof.

In certain aspects, use of the methods provided herein, *i.e.*, administration of benralizumab or an antigen-binding fragment thereof, reduces exacerbation rates within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, or within 52 weeks.

The methods provided herein can reduce exacerbation rates in COPD patients with eosinophil counts of at least 200 eosinophils/μL prior to the administration, for example by at least 30% or by about 34%.

The methods provided herein can also reduce exacerbation rates in COPD patients with eosinophil counts of at least 300 eosinophils/μL prior to the administration, for example by at least 50% or by about 57%.

The methods provided herein can also reduce exacerbation rates in COPD patients with severe or very severe COPD (as defined by GOLD), for example by at least 40% or by about 47%.

The methods provided herein can reduce "annual exacerbation rates" in COPD patients. In assessing "annual COPD exacerbation rates," a COPD exacerbation is defined as symptomatic worsening of COPD requiring:

> a. Use of systemic corticosteroids for at least 3 days (a single depot injectable dose of corticosteroids is considered equivalent to a 3-day course of systemic corticosteroids; and/or
> b. Use of antibiotics; and/or
> c. An inpatient hospitalization due to COPD.

The methods provided herein can reduce the time to a first COPD exacerbation after a first administration of benralizumab or an antigen-binding fragment thereof as compared to after a first administration of placebo.

In some instances, administration of benralizumab or an antigen-binding fragment thereof decreases the likelihood of a COPD exacerbation (e.g., within 52 weeks of a first administration of benralizumab or an antigen-binding fragment thereof) as compared to the likelihood of a COPD exacerbation after treatment with placebo.

In some instances, administration of benralizumab or an antigen-binding fragment thereof decreases the annual rate of COPD exacerbations that are associated with an emergency room or hospitalization as compared to administration of placebo.

In certain instances, administration of benralizumab or an antigen-binding fragment thereof improves the pulmonary function in a COPD patient, for example, as measured by forced expiratory volume in one second ($FEV_1$) or forced vital capacity.

The methods provided herein can increase forced expiratory volume in one second ($FEV_1$) in COPD patients. An increase can be measured based on the expected $FEV_1$ based on a large patient population, on the $FEV_1$ measured in a control population, or on the individual patient's $FEV_1$ prior to administration. In certain aspects, use of the methods provided herein, *i.e.*, administration of benralizumab or an antigen-binding fragment thereof, can increase the $FEV_1$, as compared to the patient's baseline $FEV_1$. In some embodiments, the increased $FEV_1$ is pre-bronchodilator $FEV_1$. In some embodiments, the increased $FEV_1$ is post-bronchodilator $FEV_1$. In some embodiments, the increased $FEV_1$ is pre-bronchodilator $FEV_1$ and post-bronchodilator $FEV_1$. The $FEV_1$ (e.g., the pre-bronchodilator and/or post-bronchodilator $FEV_1$) can be increased, for example, within a year from the first administration of benralizumab or the antigen-binding fragment thereof.

A "bronchodilator," as used herein, refers to any drug that widens or dilates the bronchi and bronchioles or air passages of the lungs, decreases resistance in the respiratory airway, and/or eases breathing by relaxing bronchial smooth muscle. For example, bronchodilators include short- and long- acting β2-agonists such as albuterol/salbutamol and other drugs commonly used to treat asthma.

In certain aspects, the methods provided herein can increase $FEV_1$ by at least 5% or by at least 10%. In certain aspects, the methods provided herein can increase $FEV_1$ by about 12%. In certain aspects, the methods provided herein can increase pre-bronchodilator $FEV_1$ by at least 5% or by at least 10%. In certain aspects, the methods provided herein can increase pre-bronchodilator $FEV_1$ by about 12%.

In certain aspects, the methods provided herein can increase $FEV_1$ by at least 5%. In certain aspects, the methods provided herein can increase $FEV_1$ by about 7%. In certain aspects, the methods provided herein can increase post-bronchodilator $FEV_1$ by at least 5%. In certain aspects, the methods provided herein can increase post-bronchodi-

lator $FEV_1$ by about 7%.

In certain aspects, the methods provided herein can increase pre-bronchodilator and post-bronchodilator $FEV_1$ by at least 5%. In certain aspects, the methods provided herein can increase can increase pre-bronchodilator by at least 10% and post-bronchodilator $FEV_1$ by at least 5%. In certain aspects, the methods provided herein can increase pre-bronchodilator $FEV_1$ by about 12% and post-bronchodilator $FEV_1$ by about 7%.

As provided herein, administration of benralizumab or the antigen-binding fragment thereof can also increase the percent predicted $FEV_1$ in COPD patients e.g., pre-bronchodilator and/or post-bronchodilator. By way of example, the percent predicted $FEV_1$ can increase by about 3.0, about 3.5, about 4.0, or about 4.5.

The methods provided herein can increase $FEV_1$ in COPD patients with blood eosinophil counts of at least 200 eosinophils/$\mu$L, or in patients receiving corticosteroids (e.g., inhaled corticosteroids (ICS), long-acting $\beta$-agonists (LABA) (e.g., long-acting $\beta$2-agonists), and tiotropium. In certain aspects, the methods provided herein can increase $FEV_1$ in such patients by at least 10% or by at least 15%. In certain aspects, the methods provided herein can increase pre-bronchodilator $FEV_1$ in such patients by at least 10% or by at least 15%. In certain aspects, the methods provided herein can increase post-bronchodilator $FEV_1$ in such patients by about 10%. In certain aspects, the methods provided herein can increase pre-bronchodilator $FEV_1$ and post-bronchodilator $FEV_1$ in such patients by at least 10%. In certain aspects, the methods provided herein can increase pre-bronchodilator $FEV_1$ in such patients by at least 15% and post-bronchodilator $FEV_1$ in such patients by at least 10%.

The methods provided herein can increase $FEV_1$ in COPD patients with blood eosinophil counts of at least 300 eosinophils/$\mu$L or in COPD patients with severe or very severe COPD as defined by the Global Initiative for Chronic Obstructive Lung Disease (GOLD). In certain aspects, the methods provided herein can increase $FEV_1$ in such patients by at least 15% or by at least 20%. In certain aspects, the methods provided herein can increase pre-bronchodilator $FEV_1$ in such patients by at least 15% or by at least 20%. In certain aspects, the methods provided herein can increase post-bronchodilator $FEV_1$ in such patients by about 15%. In certain aspects, the methods provided herein can increase pre-bronchodilator $FEV_1$ and post-bronchodilator $FEV_1$ in such patients by at least 15%. In certain aspects, the methods provided herein can increase pre-bronchodilator $FEV_1$ in such patients by at least 20% and post-bronchodilator $FEV_1$ in such patients by at least 15%.

In certain aspects, use of the methods provided herein, *i.e.*, administration of benralizumab or an antigen-binding fragment thereof, increases the $FEV_1$ within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more. In certain aspects, administration of benralizumab or an antigen-binding fragment thereof improves $FEV_1$ within 52 weeks of a first administration of the benralizumab or antigen-binding fragment thereof. Use of the methods provided herein can increase $FEV_1$ by at least 0.05 L, at least 0.1 L, at least 0.13 L, at least 0.15 L, at least 0.20 L, at least 0.21 L, at least 0.22 L, at least 0.23 L, at least 0.24 L, or at least 0.25 L, at least 0.30 L, at least 0.35 L, at least 0.40 L, at least 0.45 L, or at least 0.50 L over the 56-week period.

The methods provided herein can increase forced vital capacity (FVC) in COPD patients. An increase can be measured based on the expected FVC based on a large patient population, on the FVC measured in a control population, or on the individual patient's FVC prior to administration. In certain aspects, use of the methods provided herein, *i.e.*, administration of benralizumab or an antigen-binding fragment thereof, can increase the FVC, as compared to the patient's baseline FVC. In some embodiments, the increased FVC is pre-bronchodilator FVC. In some embodiments, the increased FVC is post-bronchodilator FVC. In some embodiments, the increased FVC is pre-bronchodilator FVC and post-bronchodilator FVC. The FVC (e.g., the pre-bronchodilator and/or post-bronchodilator FVC) can be increased, for example, within a year from the first administration of benralizumab or the antigen-binding fragment thereof.

In certain aspects, the methods provided herein can increase FVC by at least 3%. In certain aspects, the methods provided herein can increase pre-bronchodilator FVC by at least 2%, at least 3%., at least 5% or at least 10%. In certain aspects, the methods provided herein can increase post-bronchodilator FVC by at least 2%, at least 3%., at least 5% or at least 10%. In certain aspects, the methods provided herein can increase pre-bronchodilator and post-bronchodilator FVC by at least 2%, at least 3%., at least 5% or at least 10%. In certain aspects, use of the methods provided herein, *i.e.*, administration of benralizumab or an antigen-binding fragment thereof, increases FVC within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more.

In certain instances, administration of benralizumab or an antigen-binding fragment thereof improves respiratory symptoms in a COPD patient, for example, as measured by the Baseline/Transitional Dyspnea Index (BDI/TDI) and/or the Exacerbations of Chronic Pulmonary Disease Tool - Respiratory Symptoms (E-RS).

Provided herein are also methods for improving respiratory symptoms as measured by the Baseline/Transitional Dyspnea Index (TDI). For example, administration of benralizumab or an antigen-binding fragment thereof can improve (increase) a COPD patient's BDI score by at least 1, at least 2, or at least 3 and/or result in a positive TDI score. The BDI/TDI score can be improved, for example, within a year from the first administration of benralizumab or the antigen-binding fragment thereof.

In certain aspects, use of the methods provided herein, *i.e.*, administration of benralizumab or an antigen-binding fragment thereof, improves a BDI/TDI score within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more.

Provided herein are also methods for improving respiratory symptoms as measured by the Exacerbations of Chronic Pulmonary Disease Tool - Respiratory Symptoms (E-RS). For example, administration of benralizumab or an antigen-binding fragment thereof can improve (decrease) a COPD patient's E-RS score by least 3, at least 4, at least 6, at least 7, at least 8, at least 9, or at least 10. The E-RS score can be improved, for example, within a year from the first administration of benralizumab or the antigen-binding fragment thereof.

In certain aspects, use of the methods provided herein, *i.e.*, administration of benralizumab or an antigen-binding fragment thereof, improves a E-RS score within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more.

In certain instances, administration of benralizumab or an antigen-binding fragment thereof improves the health status and/or health-related quality of life in a COPD patient, for example, as measured by the Saint George's Respiratory Questionnaire (SGRQ), the COPD-Specific Saint George's Respiratory Questionnaire (SGRQ-C), and/or the COPD assessment tool (CAT).

Provided herein are methods for improving COPD symptoms, e.g., as assessed using a COPD questionnaire such as the Saint George's Respiratory Questionnaire (SGRQ). For example, administration of benralizumab or an antigen-binding fragment thereof can improve a patient's SGRQ score by at least 3, at least 4, at least 6, at least 7, at least 8, at least 9, or at least 10. The SGRQ score can be improved, for example, within a year from the first administration of benralizumab or the antigen-binding fragment thereof.

In certain aspects, use of the methods provided herein, *i.e.*, administration of benralizumab or an antigen-binding fragment thereof, improves a SGRQ score within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more. In certain aspects, administration of benralizumab or an antigen-binding fragment thereof improves an SGRQ score within 52 weeks of a first administration of the benralizumab or antigen-binding fragment thereof.

Provided herein are also methods for improving COPD symptoms, e.g., as assessed using a COPD questionnaire such as the COPD-Specific Saint George's Respiratory Questionnaire (SGRQ-C). For example, administration of benralizumab or an antigen-binding fragment thereof can improve a COPD patient's SGRQ-C (symptom) score by at least 3, at least 4, at least 6, at least 7, at least 8, at least 9, or at least 10. The SGRQ-C (symptom) score can be improved, for example, within a year from the first administration of benralizumab or the antigen-binding fragment thereof.

In certain aspects, use of the methods provided herein, *i.e.*, administration of benralizumab or an antigen-binding fragment thereof, improves a SGRQ-C (symptom) score within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more.

Provided herein are also methods for improving COPD symptoms, e.g., as assessed using the COPD assessment tool (CAT). For example, administration of benralizumab or an antigen-binding fragment thereof can improve (decrease) a COPD patient's CAT score by least 3, at least 4, at least 6, at least 7, at least 8, at least 9, or at least 10. The CAT score can be improved (decreased), for example, within a year from the first administration of benralizumab or the antigen-binding fragment thereof.

In certain aspects, use of the methods provided herein, *i.e.*, administration of benralizumab or an antigen-binding fragment thereof, improves (decreases) a CAT score within 4 weeks, within 8 weeks, within 12 weeks, within 16 weeks, within 20 weeks, within 24 weeks, within 28 weeks, within 32 weeks, within 36 weeks, within 40 weeks, within 44 weeks, within 48 weeks, within 52 weeks, or within 56 weeks or more.

In certain aspects, use of the methods provided herein, *i.e.*, administration of benralizumab or an antigen-binding fragment thereof, reduces nocturnal awakenings.

In certain aspects, use of the methods provided herein, *i.e.*, administration of benralizumab or an antigen-binding fragment thereof, reduces the use of rescue medication.

In certain aspects, use of the methods provided herein, *i.e.*, administration of benralizumab or an antigen-binding fragment thereof, reduces the severity, frequency, and/or duration of EXACT-PRO defined events.

In certain aspects, use of the methods provided herein, *i.e.*, administration of benralizumab or an antigen-binding fragment thereof, reduces COPD-specific resource utilization. For example, administration of benralizumab or an antigen-binding fragment thereof can reduce unscheduled physician visits, unscheduled phone calls to physicians, and/or use of other COPD medications.

In certain aspects, use of the methods provided herein, i.e., administration of benralizumab or antigen-binding fragment thereof to a COPD patient, increases forced expiratory volume in one second ($FEV_1$), increases forced vital

capacity (FVC), reduces COPD exacerbation rate, and/or improves a COPD questionnaire score (e.g., the COPD control questionnaire).

In certain aspects, use of the methods provided herein, i.e., administration of benralizumab or antigen-binding fragment thereof to a COPD patient, decreases annual COPD exacerbation rate, improves SGRQ scores, and increases $FEV_1$ (e.g., in COPD patients with a baseline blood eosinophil count ≥300/μL).

In certain aspects, the COPD patient was prescribed or has been using corticosteroids (e.g., inhaled corticosteroids (ICS)), long-acting β-agonists (LABA, e.g., long-acting β2-agonists), and tiotropium prior to the administration of benralizumab or an antigen-binding fragment thereof. In certain aspects, the COPD patient is treated with corticosteroids (e.g., ICS), LABA (e.g., long-acting β2-agonists), tiotropium, and benralizumab or an antigen-binding fragment thereof. In certain aspects, the COPD patient is treated with ICS and LABA. In certain aspects, the COPD patient is treated with LABA and long-acting muscarinic antagonist (LAMA). In certain aspects, the COPD patient is treated with ICS and LABA or with LABA and LAMA. In certain aspects, the COPD patient is treated with ICS, LABA, and LAMA.

In certain aspects of the methods provided herein, the patient has a history of COPD exacerbations. In certain aspects, the history of exacerbations comprises at least one exacerbation in the year prior to the administration of benralizumab or an antigen-binding fragment thereof. In certain aspects, the patient has a forced expiratory volume ($FEV_1$) of less than 80% predicted value prior to the administration. In certain aspects, the patient has an $FEV_1$/FVC of less than 0.70 prior to the administration.

In certain aspects, the COPD patient has a particular blood eosinophil count, e.g., prior to the administration of benralizumab or an antigen-binding fragment thereof. Blood eosinophil counts can be measured, for example, using a complete blood count (CBC) with cell differential. In certain aspects, the COPD patient has a blood eosinophil count of at least 200 cells/μL prior to the administration of benralizumab or an antigen-binding fragment thereof. In certain aspects, the COPD patient has a blood eosinophil count of at least 300 cells/μL prior to the administration of benralizumab or an antigen-binding fragment thereof. In certain aspects, the patient has a blood eosinophil count of less than 150 eosinophils/μL prior to the administration of benralizumab or an antigen-binding fragment thereof. In certain aspects, the patient has a blood eosinophil count of less than 300 eosinophils/μL prior to the administration of benralizumab or an antigen-binding fragment thereof. In certain aspects, the patient has a blood eosinophil count of 150-300 eosinophils/μL prior to the administration of benralizumab or an antigen-binding fragment thereof. In certain aspects, the patient has a blood eosinophil count of 300-450 eosinophils/μL prior to the administration of benralizumab or an antigen-binding fragment thereof. In certain aspects, the patient has a blood eosinophil count of greater than 400 eosinophils/μL prior to the administration of benralizumab or an antigen-binding fragment thereof. In certain aspects, the patient has a blood eosinophil count of greater than 450 eosinophils/μL prior to the administration of benralizumab or an antigen-binding fragment thereof.

In certain aspects, the COPD patient has severe COPD has defined by Global Initiative for Chronic Obstructive Lung Disease (GOLD), i.e., GOLD III. In certain aspects, the COPD patients have very severe COPD as defined by GOLD, i.e., GOLD IV. In certain aspects, the COPD patient has severe or very severe COPD as defined by GOLD, i.e., GOLD III or IV.

Examples

EXAMPLE 1: Patients and Methods

(a) SUBJECTS

Subjects in this study were required to be 40 to 85 years of age with moderate-to-severe COPD as defined by the Global Initiative for Chronic Obstructive Lung Disease (GOLD), Global strategy for the diagnosis, management, and prevention of chronic obstructive pulmonary disease (updated 2009), i.e., GOLD II-IV. The subjects must also have had a documented history of one or more acute exacerbations of COPD (AECOPD) that required treatment with systemic corticosteroids and/or antibiotics, or hospitalization within 2-12 months prior to Day 1, but must have been clinically stable and free from an AECOPD for 8 weeks prior to Day 1. The subjects must also have had and a sputum eosinophil count of ≥ 3.0% within 12 months prior to, or at screening. The subjects must also have had a post-bronchodilator forced expiratory volume in 1 second ($FEV_1$) / forced vital capacity (FVC) < 0.70 and a post-bronchodilator $FEV_1$ < 80% predicted at screening. All subjects were current smokers or ex-smokers with a tobacco history of ≥ 10 pack-years (1 pack year = 20 cigarettes smoked per day for 1 year). Subjects receiving allergy immunotherapy must have been on a stable dose for the 90 days preceding Day 1.

Subjects were not eligible to participate if they had other significant pulmonary disease as a primary diagnosis (e.g., cystic fibrosis, bronchiectasis, alpha-1 antitrypsin deficiency, interstitial lung disease; pulmonary hypertension other than corpulmonale) or they were receiving long-term oxygen therapy (use of oxygen for a minimum of 15 hours per day) at entry into the study. Subjects were also not eligible to participate if they had a current diagnosis of asthma or had a lung volume reduction surgery with the 12 months prior to screening. Subjects were also not eligible to participate if they had significant or unstable ischemic heart disease, arrhythmia, cardiomyopathy, heart failure, or renal failure, uncontrolled hypertension, or a malignancy within the past 5 years (except adequately treated noninvasive basal cell and squamous cell carcinoma of the skin and cervical carcinoma-in-situ treated with apparent success more than 1 year prior to screening). Subjects using immunosuppressive medication, including inhaled (other than Symbicort®), topical, ocular, nasal or rectal corticosteroids or systemic steroids within 28 days before randomization (Day 1) were also not eligible to participate.

(b) DESIGN OF THE STUDY

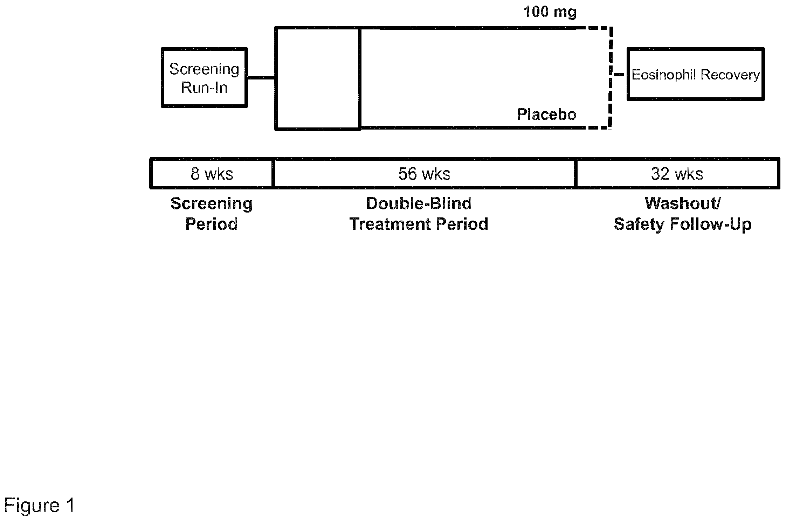

The study was a phase 2a randomized, double-blind, placebo-controlled, multicenter study in which multiple doses of benralizumab were administered subcutaneously to COPD patients. Benralizumab was administered at 100 mg doses for 48 weeks and continued to be followed for 32 weeks afterwards. The study flow diagram is shown in Figure 1.

Subjects were screened between Day -56 and Day -29. Prior to randomization, all subjects underwent a 28-day run-in period (Day -28 to Day -1), during which their current ICS and/or long-acting $\beta$-agonist combination product were replaced with Symbicort® (budesonide/formoterol fumarate) 200/6 $\mu$g/inhalation: 2 inhalations twice daily if $FEV_1$ was < 50% predicted or Spiriva® (tiotropium bromide monohydrate) 18 $\mu$g/inhalation once daily if $50\% \leq FEV1 < 80\%$ predicted. The subjects were provided with a short-acting $\beta2$-agonist for symptom relief during the study (terbutaline sulphate, Bricanyl®). Subjects who remained clinically stable during the 28-day run-in period and met eligibility criteria continued the maintenance treatment with Symbicort® or Spiriva® and could be randomized into the study to receive investigational product as an add-on therapy for 48 weeks.

A total of 101 subjects from multiple sites were randomized in a 1:1 ratio to receive either 100 mg subcutaneous (SC) benralizumab or placebo. Investigational product (benralizumab or placebo) was administered subcutaneously in an outpatient setting every 28 days (4 weeks) for the first 3 doses and then every 56 days (8 weeks) for the next 5 doses up to Day 337 (total 8 doses). The day of receipt of the first dose of investigational product was considered Day 1. Subjects were followed for a total of 32 additional weeks (to Day 561). Post Day 561, subjects continued until peripheral blood eosinophil counts returned to 50 cells/$\mu$L or 20% of baseline.

Baseline measurements at screening included evaluation of disease activity; pulmonary function tests (forced vital capacity (FVC), $FEV_1$); patient-reported outcomes; analysis of eosinophil-generated proteins; sputum induction for analysis to include cell count; medical assessment, and pulse oximetry. The patient reported outcomes included COPD-specific Saint George's Respiratory Questionnaire (SGRQ-C), and Chronic Respiratory Questionnaire self-administered standardized format (CRQ-SAS).

During the course of the study, evaluations included assessments of disease activity; pulmonary function testing; inflammation markers associated with COPD and the acute phase response; assessment of exacerbations; use of concomitant medications; and patient-reported outcomes (SGRQ-C, CRQ-SAS). Not all evaluations were done at each visit. In the event of a moderate-to-severe exacerbation, additional evaluations were performed.

(i) Assessment of Acute Exacerbations of COPD

The severity of an exacerbation of COPD was defined as follows. Mild exacerbations require treatment with an increase in usual therapy, e.g., increase use of short acting bronchodilators. Moderate exacerbations require treatment with systemic corticosteroids, and or antibiotics. Severe exacerbations require hospitalization. When symptoms changed or exacerbations occurred, subjects were instructed to contact the investigator immediately and report to the clinic as soon as possible (within three days) if there was no satisfactory relief.

On contact from the subject, the study site confirmed the exacerbation onset by administering a brief exacerbation assessment based on the Anthonisen definition of an AECOPD: worsening of 2 or more major symptoms (dyspnea, sputum volume, and sputum purulence) or worsening of any one major symptom together with any one of the following minor symptoms: sore throat, colds (nasal discharge and/or nasal congestion), fever without other cause, and increased cough or wheeze for at least 2 consecutive days. Anthonisen et al., Ann. Int. Med. 106:196-204 (1987).

Duration of an AECOPD was defined as the length of time (days) between day of onset and recovery. Recovery is the point at which a subject experiences sustained improvement in their event, with a decrease in EXACT score $\geq 9$ points from the maximum observed value (MOV) on any subsequent day during the observational period. The first of the 7 consecutive days of improvement is designated the first day of recovery.

A relapse of AECOPD was defined as a worsening of AECOPD symptoms after an initial improvement but prior to achieving a stable chronic COPD treatment regimen for a minimum of 14 days and requiring re-treatment with systemic corticosteroids, or hospitalization. For the purposes of this study, a relapse of AECOPD was not considered to be the same as a new episode of AECOPD as regards to the analysis of the rate of AECOPD. Aaron et al., Chest 121:688-96 (2002). It should be noted that a subject may not return to their previous level of function after resolution of an episode of AECOPD.

Besides subject-reported AECOPD episodes, frequency of AECOPD was also assessed using the EXACT-PRO score change for unreported AECOPD episodes defined as: an increase of 12 points above the subject's mean baseline for 2 consecutive days or an increase of 9 points above subject mean baseline for 3 consecutive days.

(ii) Pulmonary Function Tests

COPD evaluations were also assessed via airflow limitation (spirometry with forced vital capacity (FVC), forced expiratory volume in 1 second ($FEV_1$), and $FEV_1/FVC$). Spirometry pre- and post- albuterol/salbutamol (4 puffs) or equivalent dose of other inhaled short acting β2-agonist were performed at study sites by the investigator or qualified designee at designated visits. Post-bronchodilator assessments were generally performed within 10-30 minutes after albuterol/salbutamol. Prior to spirometry testing, subjects were required to withhold short-acting β2-agonists for at least 6 hours (including reliever medication), long-acting β2-agonists and caffeinated food products including caffeinated drinks for at least 12 hours, and any medication containing ephedrine/pseudo-ephedrine for at least 48 hours. Subjects were also asked not to smoke within 1 hour, consume alcohol within 4 hours, exercise vigorously within 2 hours, or consume large meals within 2 hours of the spirometry testing.

Multiple forced expiratory efforts (at least 3 but no more than 8) were performed for each office spirometry session, and the 2 best efforts that meet American Thoracic Society (ATS) or European Respiratory Society (ERS) acceptability and reproducibility criteria were recorded. The best efforts will be based on the highest $FEV_1$. The maximum $FEV_1$ of the 2 best efforts was used for the analysis. Both the absolute measurement (for $FEV_1$ and FVC) and the percentage of predicted normal value were recorded. The highest FVC was also reported regardless of the effort in which it occurred (even if the effort did not result in the highest $FEV_1$). Nose clips were used for office spirometry.

Office spirometry was performed on Day -56, Day 1, Day 29, Day 57, Day 113, Day 169, Day 225, Day 281, Day 337, and Day 393. Additional office spirometry is performed on Day 477 and Day 561.

(iii) COPD-Specific Saint George's Respiratory Questionnaire (SGRQ-C)

Overall health status of subjects with airway obstructive diseases was assessed with the COPD-specific Saint George's Respiratory Questionnaire (SGRQ-C), a 40-item patient-reported outcome. Jones et al., Respir. Med. 85:Suppl B:25-31 (1991) and Meguro et al., Chest 132:456-63 (2007). Responses included yes or no, and 3- to 5-point scales assessing the impact of symptoms, activities, and impact on daily life. Total scores and domain scores (symptoms, activities, and impact on daily life) were scored from 0-100, where lower scores indicate better health status. A 4-point change in total score has been demonstrated to be a clinically meaningful change, while an 8-point change and a 12-point change have been interpreted as a moderate and large change in health status, respectively.

SGRQ-C assessments were performed at Day -56, Day 1, Day 29, Day 57, Day 113, Day 169, Day 225, Day 281, Day 337, and Day 393. SGRQ-C assessments are also performed on Day 477 and Day 561.

(iv) Chronic Respiratory Questionnaire (CRQ)

The chronic respiratory questionnaire (CRQ), a widely used measure of health-related quality of life (HRQOL) in patients with chronic airflow limitation, includes an individualized dyspnea domain. Guyatt et al., Thorax 42:773-8 (1987). Subjects identify five important activities, and report the degree of dyspnea on a 7-point scale. The original CRQ was designed to be interviewer administered questionnaire. The patient self-administered standard version of CRQ (CRQ-SAS) has been validated and was being administered in this study. Williams et al, Thorax 56:954-9 (2001). The CRQ and the subsequent CRQ-SAS are made up of four dimensions relating to dyspnea, emotional function, fatigue, and mastery. There are 20 questions in total and for every question there is a range of responses that score from 1 to 7. The dimensions include fatigue, emotional function, and mastery, which are scored from 1 to 7. In each dimension the lower the score, the greater the degree of dysfunction.

CRQ-SAS assessments were performed at Day -56, Day 1, Day 29, Day 57, Day 113, Day 169, Day 225, Day 281, Day 337, and Day 393. CRQ-SAS assessments are also performed on Day 477 and Day 561.

(v) Exacerbation Symptom Assessment Based on Anthonisen Definition

Once subjects contacted the study site due to an increase in COPD symptoms that are not relieved by an increase in Bricanyl® usage, the study site assessed the subjects' exacerbation symptoms using the major and minor symptoms based on the Anthonisen definition. Major symptoms include dyspnea, sputum purulence, and sputum volume, and minor symptoms include cough/wheeze, fever, sore throat, and cold (nasal discharge/congestion). Anthonisen et al., Ann. Int. Med 106:196-204 (1987). Dyspnea, sputum purulence and volume, and cough/wheeze were evaluated relative to their usual state while others were evaluated based on their absence or presence for the past 2 days. Subjects rated their symptoms using a 3-point scale.

A COPD exacerbation was defined as worsening of two or more major symptoms or one major and one minor symptom for two or more consecutive days. A study investigator or coordinator confirmed subjects' exacerbations.

Assessments of AECOPD based on the Anthonisen definition were performed at Day -56, Day 1, Day 29, Day 57, Day 113, Day 169, Day 225, Day 281, Day 337, and Day 393. Assessments of AECOPD based on the Anthonisen definition are also performed on Day 341, Day 477, and Day 561.

(c) SAFETY ASSESSMENTS

Adverse events were monitored following administration of placebo or benralizumab. Other assessments included physical examination, vital sign monitoring, and laboratory measurements including hematology, chemistry, and urinalysis.

EXAMPLE 2: Results

(a) ENROLLMENT AND BASELINE CHARACTERISTICS

The Intent-to-Treat (ITT) population includes all subjects who were randomized into the study. The treatment group was assigned according to an initial randomization, regardless of whether subjects received any investigational product or received an investigational product different from that to which they were randomized. Of the 101 subjects in the ITT population, 50 received placebo, and 51 received benralizumab (100 mg).

The baseline characteristics of the ITT population are provided in Table 1 below.

**Table 1: Demographics for ITT Population**

|  |  | Placebo (N=50) | Benralizumab 100 mg (N=51) | Total (N=101) |
|---|---|---|---|---|
| Age (years) | Mean (SD) | 64.6 (7.5) | 62.9 (8.2) | 63.7 (7.9) |
| Gender | Male<br>Female | 29 (58.0%)<br>21 (42.0%) | 35 (68.6%)<br>16 (31.4%) | 64 (63.4%)<br>37 (36.6%) |
| Weight (kg) | Mean (SD) | 75.2 (13.5) | 76.1 (18.0) | 75.7 (15.8) |
| Height (cm) | Mean (SD) | 168.8 (9.6) | 168.8 (8.4) | 168.8 (9.0) |
| BMI (kg/m^2) | Mean (SD) | 26.5 (4.8) | 26.6 (5.6) | 26.6 (5.2) |
| $FEV_1$ Pre-bronch (L) | Mean (SD) | 1.412 (0.568) | 1.305 (0.546) |  |
| $FEV_1$ Post-bronch (L) | Mean (SD) | 1.529 (0.575) | 1.472 (0.545) |  |

The Per-Protocol (PP) population includes all subjects who had no major protocol violations, have received at least 6 of the 8 total doses (at least 2 of the first 2 doses on Days 1 and 29, and at least 4 of the last 6 doses on Days 57, 113, 169, 225, 281, and 337) of investigational product, and have completed the study through Day 393. The PP population was identified prior to database lock (i.e., prior to restricting access to the clinical study database after known data processing activities are complete). Of the 84 subjects in the PP population, 44 received placebo and 40 received benralizumab (100 mg).

(b) EFFICACY

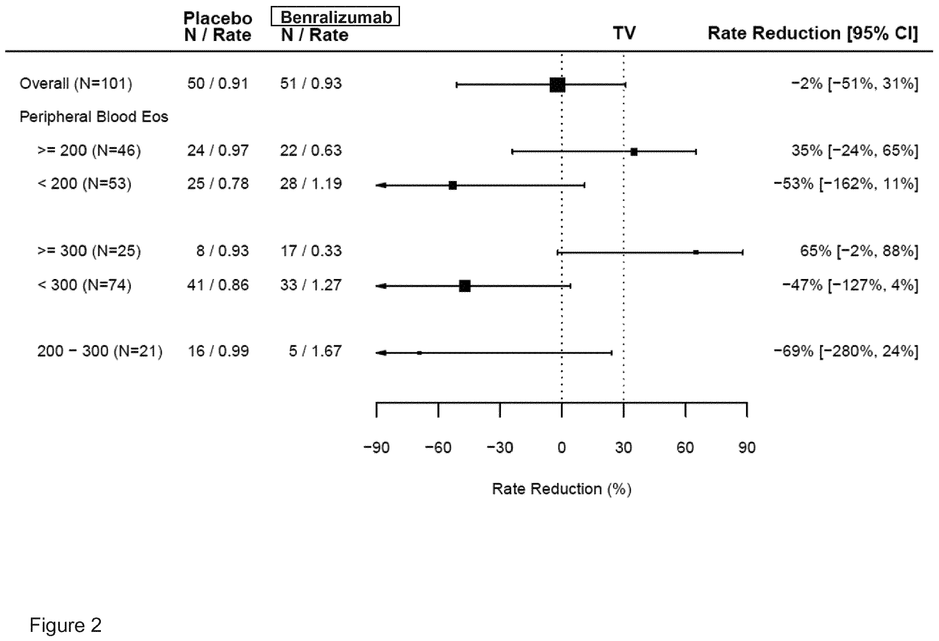

The effects of administration of benralizumab on moderate-to-severe acute exacerbations of COPD (AECOPD) in various populations are shown in Table 2 below and in Figure 2.

Table 2: Severe-to-Moderate AECOPD Rate Through Day 393

| Population | Placebo (N) | Benralizumab (N) | Rate Reduction | P-value |
|---|---|---|---|---|
| PP Population | 0.97 (44) | 0.98 (40) | 0.0 % | 0.913 |
| PP Population with $\geq$ 200 cells/$\mu$L | 1.11 (21) | 0.73 (19) | 34% | 0.199 |
| PP Population with $\geq$ 300 cells/$\mu$L | 0.93 (8) | 0.40 (14) | 57% | 0.197 |

(continued)

| Population | Placebo (N) | Benralizumab (N) | Rate Reduction | P-value |
|---|---|---|---|---|
| PP Population, Gold III and IV | 1.39 (16) | 0.88 (20) | 37% | 0.103 |

In the PP population, 9 of the subjects who were Gold III or IV and received placebo had $\geq$ 200 cells/$\mu$L and 4 of the subjects who were Gold III or IV and received placebo had $\geq$ 300 cells/$\mu$L. In the PP population, 10 of the subjects who were Gold III and IV and received benralizumab (100 mg) had $\geq$ 200 cells/$\mu$L, and 7 of the subjects who were Gold III and IV and received benralizumab (100 mg) had $\geq$ 300 cells/$\mu$L.

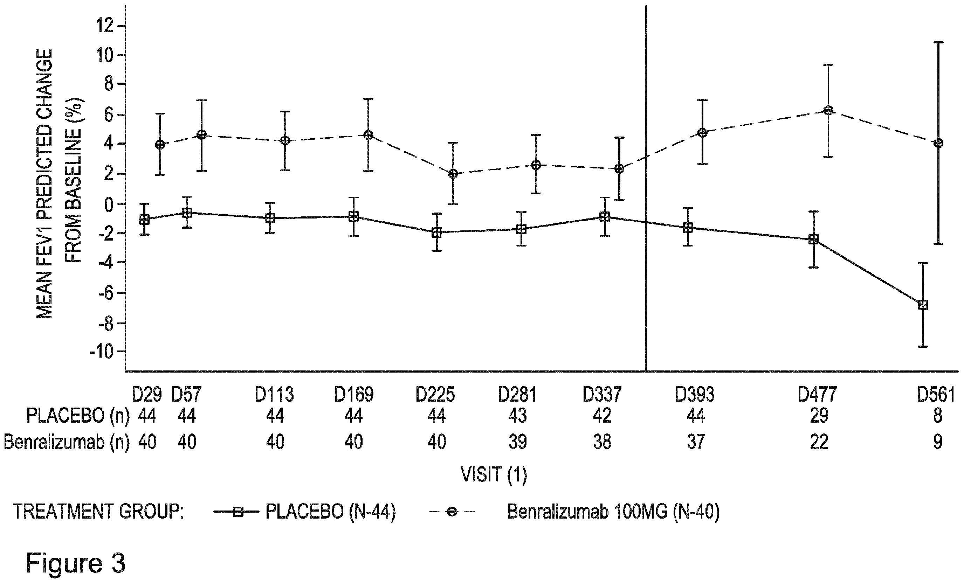

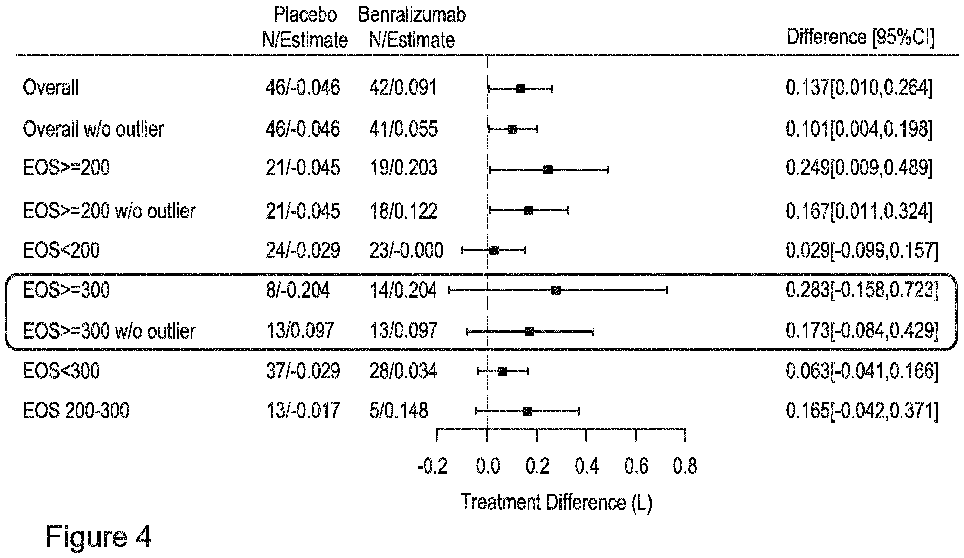

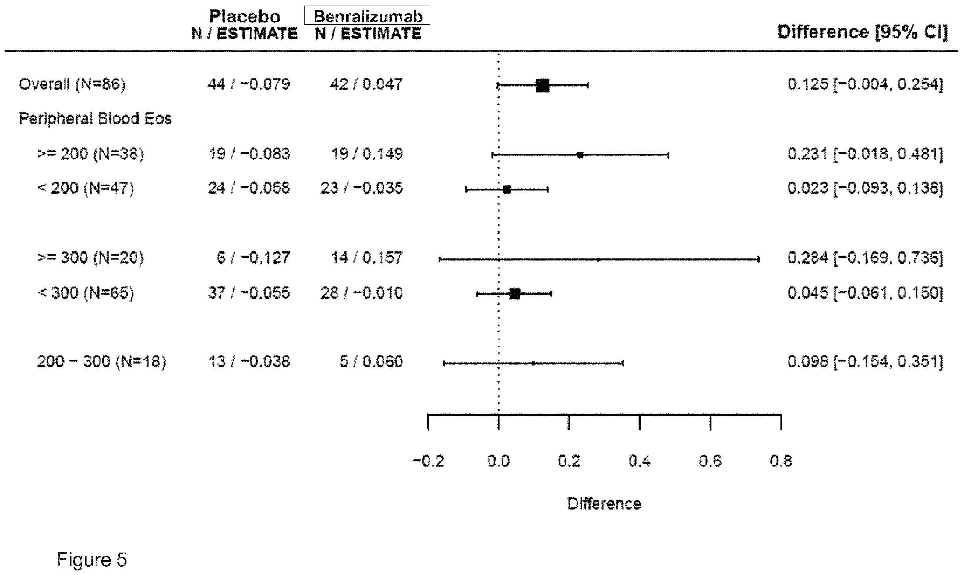

The effects of administration of benralizumab on $FEV_1$ on various populations are shown in Tables 3-8 below and in Figure 3-5.

Table 3: $FEV_1$ (L) Through Day 393 in PP Population

| | Placebo (N=44) | Benralizumab (100 mg) (N=40) | P-value |
|---|---|---|---|
| *Pre-Bronchodilator* | | | |
| Baseline Mean | 1.438 | 1.400 | |
| Day 393 Mean | 1.380 | 1.528 | |
| Mean Change from Baseline | -0.058 | 0.128 | 0.012 |
| Mean % Change from Baseline | -1.70% | 12.13% | 0.008 |
| Median Change from Baseline | -0.05 | 0.1 | |
| Median % Change from Baseline | -3.13% | 8.57% | |
| *Post- Bronchodilator* | | | |
| Baseline Mean | 1.586 | 1.565 | |
| Day 393 Mean | 1.504 | 1.656 | |
| Mean Change from Baseline | -0.082 | 0.091 | 0.014 |
| Mean % Change from Baseline | -3.7% | 7.47% | 0.015 |
| Median Change from Baseline | -0.045 | 0.000 | |
| Median % Change from Baseline | -1.87% | 0.91% | |

Table 4: $FEV_1$ (L) Through Day 393 in PP Population with $\geq$ 200 Cells/$\mu$L

| | Placebo (N=21) | Benralizumab (100 mg) (N=19) | P-value |
|---|---|---|---|
| *Pre-Bronchodilator* | | | |
| Baseline Mean | 1.319 | 1.475 | |
| Day 393 Mean | 1.280 | 1.696 | |
| Mean Change from Baseline | -0.039 | 0.211 | 0.120 |
| Mean % Change from Baseline | -2.96 | 14.98 | |
| Median Change from Baseline | -0.030 | 0.130 | |
| Median% Change from Baseline | -2.27 | 8.81 | |
| *Post- Bronchodilator* | | | |
| Baseline Mean | 1.503 | 1.605 | |
| Day 393 Mean | 1.427 | 1.800 | |
| Mean Change from Baseline | -0.076 | 0.175 | 0.030 |
| Mean % Change from Baseline | -0.506 | 12.15 | |

(continued)

| Post- Bronchodilator | | | |
|---|---|---|---|
| Median Change from Baseline | -0.030 | 0.070 | |
| Median % Change from Baseline | -1.99 | 4.36 | |

Table 5: $FEV_1$ (L) Through Day 393 in PP Population with $\geq$ 300 Cells/$\mu$L

| | Placebo (N=12) | Benralizumab (100 mg) (N=16) | P-value |
|---|---|---|---|
| Pre-Bronchodilator | | | |
| Baseline Mean | 1.665 | 1.587 | |
| Day 393 Mean | 1.571 | 1.843 | |
| Mean Change from Baseline | -0.094 | 0.257 | 0.163 |
| Mean % Change from Baseline | -1.25 | 19.88 | 0.207 |
| Median Change from Baseline | -0.110 | 0.125 | |
| Median% Change from Baseline | --5.42 | 10.96 | |
| Post- Bronchodilator | | | |
| Baseline Mean | 2.098 | 1.720 | |
| Day 393 Mean | 1.927 | 1.975 | |
| Mean Change from Baseline | -0.171 | 0.255 | 0.206 |
| Mean % Change from Baseline | -6.80 | 17.76 | 0.260 |
| Median Change from Baseline | -0.080 | 0.100 | |
| Median % Change from Baseline | -3.02 | 6.71 | |

Table 6: $FEV_1$ (L) Through Day 393 in PP Population that are Gold III or IV

| | Placebo (N=16) | Benralizumab (100 mg) (N=20) | P-value |
|---|---|---|---|
| Pre-Bronchodilator | | | |
| Baseline Mean | 0.950 | 0.989 | |
| Day 393 Mean | 0.989 | 1.234 | |
| Mean Change from Baseline | 0.039 | 0.245 | 0.116 |
| Mean % Change from Baseline | 4.91 | 21.53 | 0.092 |
| Median Change from Baseline | 0.015 | 0.140 | |
| Median% Change from Baseline | 1.15 | 12.61 | |
| Post- Bronchodilator | | | |
| Baseline Mean | 1.064 | 1.116 | |
| Day 393 Mean | 1.071 | 1.318 | |
| Mean Change from Baseline | 0.007 | 0.201 | 0.199 |
| Mean % Change from Baseline | 1.18 | 15.96 | 0.165 |
| Median Change from Baseline | 0.000 | 0.115 | |
| Median % Change from Baseline | 0.44 | 10.42 | |

Table 7: FEV$_1$ (L) Through Day 393 in Patients Receiving ICS, LABA, and Tiotropium

| | Placebo (N=26) | Benralizumab (100 mg) (N=22) | P-value |
|---|---|---|---|
| Pre- Bronchodilator | | | |
| Baseline Mean | 1.149 | 1.096 | |
| Day 393 Mean | 1.115 | 1.328 | |
| Mean Change from Baseline | -0.033 | 0.232 | 0.026 |
| Mean % Change from Baseline | 0.45 | 19.49 | 0.026 |
| Median Change from Baseline | -0.020 | 0.140 | |
| Median% Change from Baseline | -2.07 | 12.61 | |
| *Post-Bronchodilator* | | | |
| Baseline Mean | 1.301 | 1.282 | |
| Day 393 Mean | 1.216 | 1.434 | |
| Mean Change from Baseline | -0.085 | 0.153 | 0.05 |
| Mean % Change from Baseline | -3.61 | 12.07 | 0.056 |
| Median Change from Baseline | -0.020 | 0.000 | |
| Median % Change from Baseline | -2.41 | 1.04 | |

Table 8: FEV$_1$ (L) Through Day 393 in Patients Receiving ICS/LABA or Tiotropium

| | Placebo (N=20) | Benralizumab (100 mg) (N=18) | P-value |
|---|---|---|---|
| Pre-Bronchodilator | | | |
| Baseline Mean | 1.790 | 1.658 | |
| Day 393 Mean | 1.737 | 1.709 | |
| Mean Change from Baseline | -0.052 | 0.051 | 0.303 |
| Mean % Change from Baseline | -1.65 | 5.99 | 0.282 |
| Median Change from Baseline | -0.055 | 0.060 | |
| Median% Change from Baseline | -3.13 | 3.06 | |
| *Post-Bronchodilator* | | | |
| Baseline Mean | 1.906 | 1.791 | |
| Day 393 Mean | 1.854 | 1.819 | |
| Mean Change from Baseline | -0.052 | 0.029 | 0.293 |
| Mean % Change from Baseline | -2.03 | 2.79 | 0.247 |
| Median Change from Baseline | -0.025 | 0.010 | |
| Median % Change from Baseline | -1.26 | 0.91 | |

The effects of administration of benralizumab on percentage of predicted FEV$_1$ are provided in Table 9 below.

Table 9: FEV$_1$ % Predicted Through Day 393 in PP Population

| | Placebo (N=44) | Benralizumab (100 mg) (N=40) | P-value |
|---|---|---|---|
| *Pre-Bronchodilator* | | | |
| Baseline Mean | 49.97 | 46.80 | |

(continued)

|  | Placebo (N=44) | Benralizumab (100 mg) (N=40) | P-value |
|---|---|---|---|
| **Pre-Bronchodilator** | | | |
| Day 393 Mean | 48.36 | 51.62 | |
| Change from Baseline | -1.61 | 6.18 | 0.014 |
| **Post-Bronchodilator** | | | |
| Baseline Mean | 55.03 | 52.39 | |
| Day 393 Mean | 52.57 | 55.66 | |
| Change from Baseline | -2.46 | 3.27 | 0.018 |

The effects of administration of benralizumab on FVC and percentage of predicted FVC are provided in Tables 10 and 11 below.

Table 10: FVC (L) Through Day 393 in PP Population

|  | Placebo (N=50) | Benralizumab (100 mg) (N=51) | P-value |
|---|---|---|---|
| **Pre-Bronchodilator** | | | |
| Baseline Mean | 2.931 | 2.876 | |
| Day 393 Mean | 2.834 | 2.953 | |
| Change from Baseline | -0.097 | 0.076 | 0.083 |
| % Change from Baseline | -2.32% | 3.84% | 0.085 |
| **Post-Bronchodilator** | | | |
| Baseline Mean | 3.116 | 3.094 | |
| Day 393 Mean | 3.005 | 3.170 | |
| Change from Baseline | -0.111 | 0.077 | 0.051 |
| % Change from Baseline | -3.35% | 3.28% | 0.049 |

Table 11: FVC % Predicted Through Day 393 in PP Population

|  | Placebo (N=44) | Benralizumab (100 mg) (N=40) | P-value |
|---|---|---|---|
| **Pre-Bronchodilator** | | | |
| Baseline Mean | 77.08 | 72.70 | |
| Day 393 Mean | 74.65 | 75.29 | |
| Change from Baseline | -2.44 | 2.59 | 0.143 |
| **Post-Bronchodilator** | | | |
| Baseline Mean | 82.44 | 78.14 | |
| Day 393 Mean | 79.11 | 80.30 | |
| Change from Baseline | -3.33 | 2.16 | 0.073 |

The effects of administration of benralizumab on $FEV_1/FVC$ are provided in Table 12 below.

Table 12: FEV$_1$/FVC (%) Through Day 393 in PP Population

|  | Placebo (N=44) | Benralizumab (100 mg) (N=40) | P-value |
|---|---|---|---|
| **Pre-Bronchodilator** |  |  |  |
| Baseline Mean | 48.84 | 48.13 |  |
| Day 393 Mean | 49.44 | 51.65 |  |
| Change from Baseline | 0.60 | 3.52 | 0.075 |
| **Post-Bronchodilator** |  |  |  |
| Baseline Mean | 51.13 | 50.42 |  |
| Day 393 Mean | 51.38 | 52.69 |  |
| Change from Baseline | 0.24 | 2.26 | 0.198 |

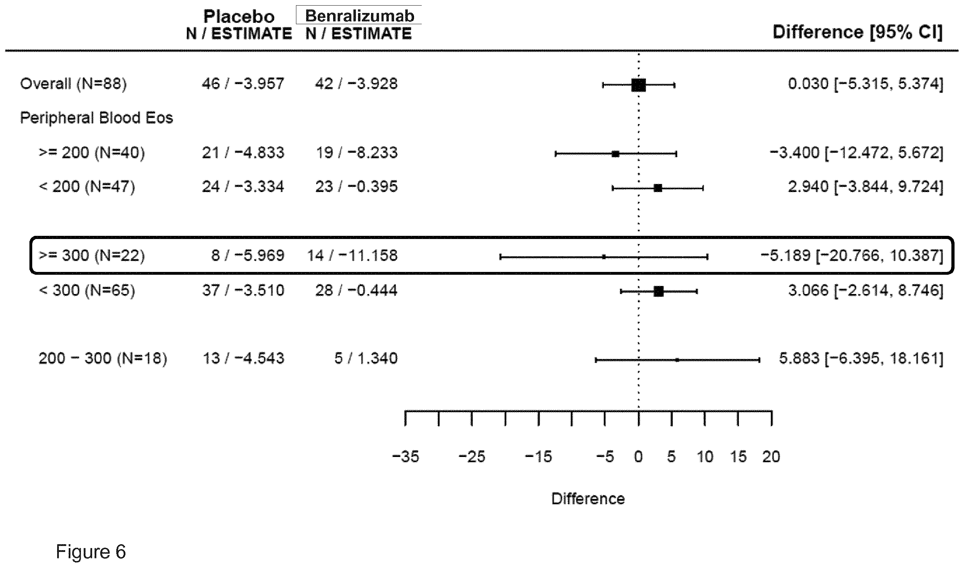

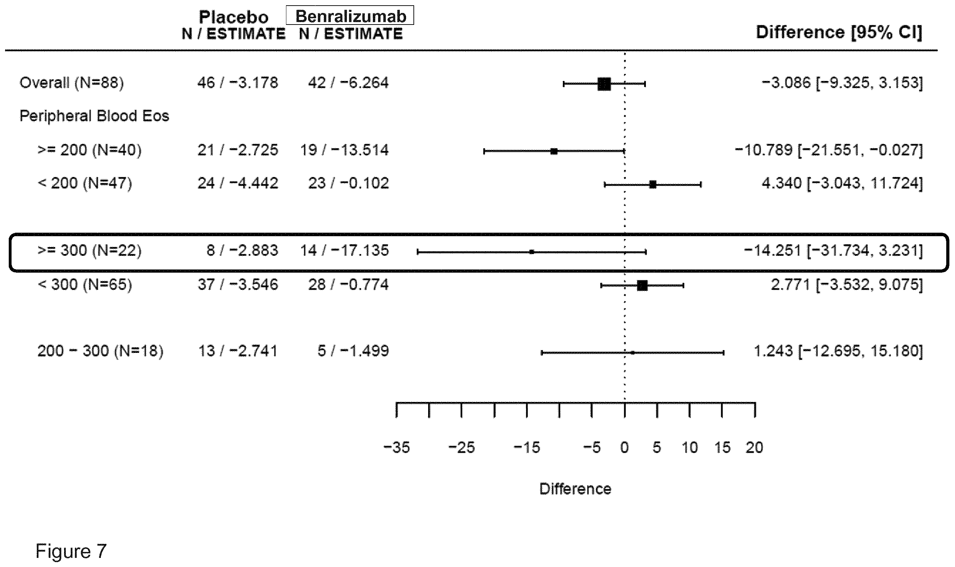

The effects of administration of benralizumab on SGRQ-C are shown in Table 13 below and in Figures 6 and 7.

Table 13: SGRQ-C Through Day 393 in PP Population [

|  | Placebo (N=44) | Benralizumab 100 mg (N=40) | Unadjusted P-value |
|---|---|---|---|
| **Total** |  |  |  |
| Baseline Mean | 48.22 | 50.63 |  |
| Day 393 Mean | 43.90 | 45.12 |  |
| Change from Baseline | -4.32 | -5.51 | 0.706[*] |
| **Symptom** |  |  |  |
| Baseline Mean | 64.15 | 65.50 |  |
| Day 393 Mean | 61.49 | 56.48 |  |
| Change from Baseline | -2.66 | -9.02 | 0.141* |
| **Activity** |  |  |  |
| Baseline Mean | 59.11 | 60.69 |  |
| Day 393 Mean | 53.55 | 56.32 |  |
| Change from Baseline | -5.56 | -4.37 | 0.790* |
| **Impact** |  |  |  |
| Baseline Mean | 36.29 | 39.55 |  |
| Day 393 Mean | 32.11 | 34.60 |  |
| Change from Baseline | -4.18 | -4.95 | 0.825* |
| * Unadjusted |  |  |  |

The effects of administration of benralizumab on CRQ-SAS are shown in Table 14 below.

Table 14: CRQ-SAS Through Day 393 in PP Population

|  | Placebo (N=44) | Benralizumab 100 mg (N=40) | P-value |
|---|---|---|---|
| **Dyspnea** |  |  |  |
| Baseline Mean | 4.95 | 4.79 |  |
| Day 393 Mean | 4.86 | 4.88 |  |
| Change from Baseline | -0.09 | 0.09 | 0.483* |

(continued)

| | Placebo (N=44) | Benralizumab 100 mg (N=40) | P-value |
|---|---|---|---|
| **Dyspnea** | | | |
| Number of Subjects with 0.5-point Change | 12 (27.3%) | 12 (32.4%) | 0.634 |
| **Fatigue** | | | |
| Baseline Mean | 4.37 | 4.05 | |
| Day 393 Mean | 4.47 | 4.16 | |
| Change from Baseline | 0.10 | 0.11 | 0.980* |
| Number of Subjects with 0.5-point Change | 16 (36.4%) | 13 (35.1%) | 1.000 |
| **Emotional Function** | | | |
| Baseline Mean | 4.84 | 4.76 | |
| Day 393 Mean | 4.98 | 4.85 | |
| Change from Baseline | 0.14 | 0.08 | 0.813* |
| Number of Subjects with 0.5-point Change | 15 (34.1%) | 10 (27.0%) | 0.630 |
| **Mastery** | | | |
| Baseline Mean | 4.90 | 4.70 | |
| Day 393 Mean | 5.11 | 4.99 | |
| Change from Baseline | 0.21 | 0.28 | 0.779* |
| Number of Subjects with 0.5-point Change | 20 (45.5%) | 14 (37.8%) | 0.508 |
| * Unadjusted | | | |

(c) SAFETY

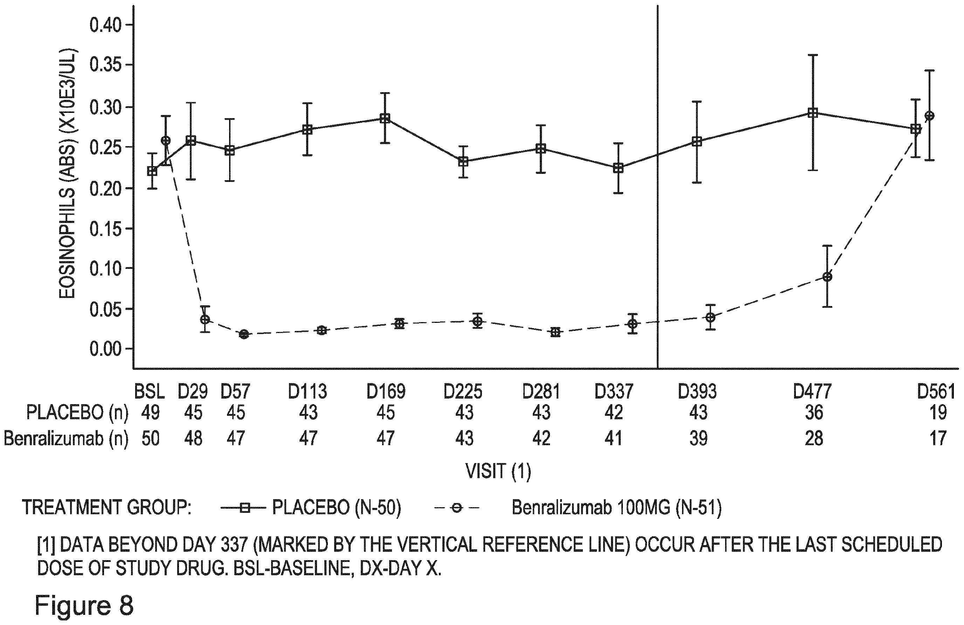

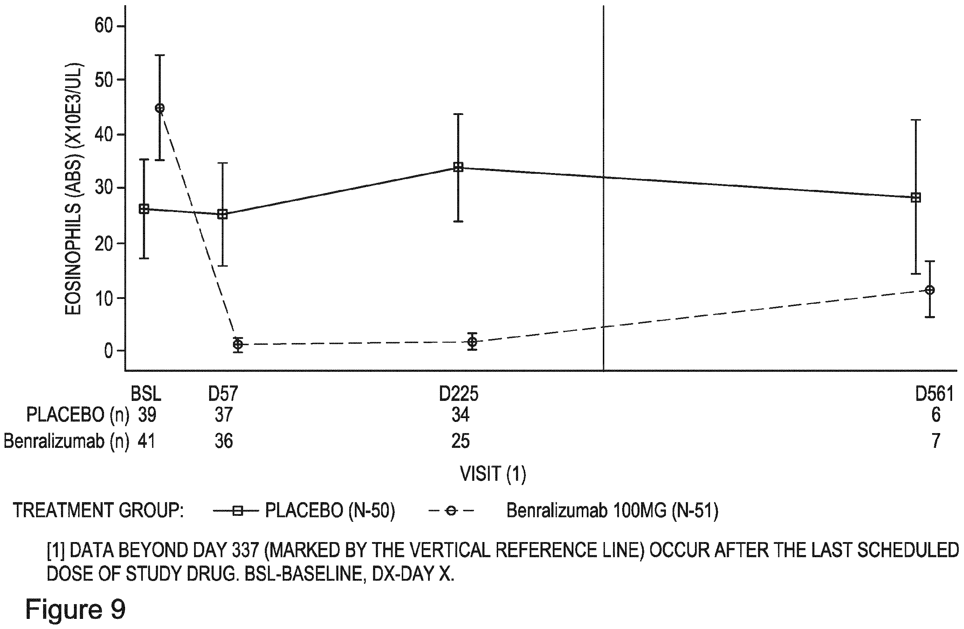

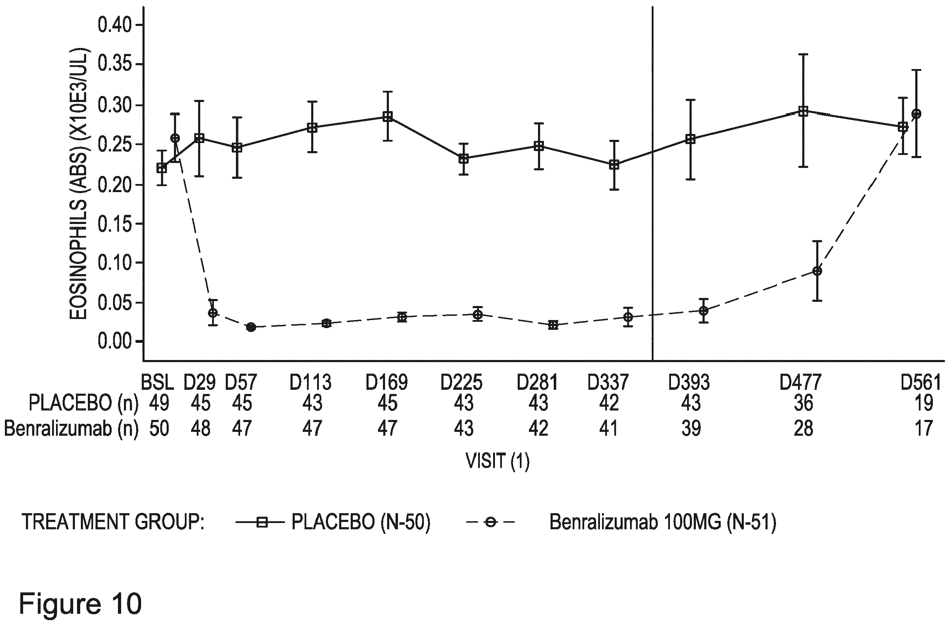

The Safety population includes all subjects who receive at least one dose of investigational product. Of the 101 subjects in the Safety population, 50 received placebo, and 51 received benralizumab (100 mg). A summary of the severe adverse events (SAEs) is shown in Table 15. In addition, the eosinophil and basophil counts over time are shown in Figures 8-10.

Table 15: Severe Adverse Events

| SAE Criteria | Placebo (N=50) | Benralizumab 100 mg (N=51) | Total (N=101) |
|---|---|---|---|
| Total Number Of Events | 13 | 22 | 35 |
| Total Subjects Reporting One Or More Events | 9 (18%) | 14 (27.5%) | 23 (22.8%) |
| Death | 0 (0.0%) | 2 (3.9%) | 2 (2.0%) |
| Life-threatening | 1 (2.0%) | 2 (3.9%) | 3 (3.0%) |
| Required Inpatient Hospitalization | 8 (16.0%) | 12 (23.5%) | 20 (19.8%) |
| Prolongation Of Hospitalization | 1 (2.0%) | 1 (2.0%) | 2 (2.0%) |
| Persistent Or Significant Disability/Incapacity | 0 (0.0%) | 1 (2.0%) | 1 (1.0%) |
| Important Medical Event | 0 (0.0%) | 2 (3.9%) | 2 (2.0%) |
| Congenital Anomaly/Birth Defect | 0 (0.0%) | 0 (0.0%) | 0 (0.0%) |

(d) DISCUSSION

This study demonstrates that benralizumab decreased exacerbation rates in COPD patients with $\geq 200$ eosinophils/$\mu$L (34% reduction; p = 0.199), in COPD patients with $\geq 300$ eosinophils/$\mu$L (57% reduction; p = 0.197), and in Gold III and IV (severe and very severe) COPD patients (47% reduction; p = 0.1.03). In addition, benralizumab also improved FEV$_1$ for both pre- and post-bronchodilator measurements and improved SGRQ-C symptom scores.

EXAMPLE 3: Use of Benralizumab to Decrease Annual COPD Exacerbation Rates

(a) SUBJECTS

Subjects in this study are required to be 40 to 85 years of age with a diagnosis of COPD and a post-bronchodilator FEV$_1$ < 50% of the predicted normal value.

Subjects must also have a Modified Medical Research Council (mMRC) score of ≥1. The mMRC dyspnea scale uses a simple grading system to assess a subject's level of dyspnea that consists of five statements about perceived breathlessness. It is an interviewer-administered ordinal scale on which subjects provide their dyspnea according to five grades of increasing severity (scores ranges from 0 (none) to 4 (very severe)).

Subjects must also have a history of ≥1 COPD exacerbation in the previous year. The COPD exacerbation within the preceding year (8 to 52 weeks prior to randomization) must have required treatment with systemic corticosteroids (a minimum 3-day course of an oral corticosteroid treatment or single depot corticosteroid injection), or hospitalization (defined as an inpatient stay or >24 hour stay in an observation area in the emergency department or other equivalent facility depending on the country and healthcare system). A history of an exacerbation treated exclusively with antibiotics is not considered adequate for inclusion in the study.

Subjects must also require maintenance treatment with double (ICS/LABA or LABA/LAMA) or triple (ICS/LABA/LAMA) therapy.

Subjects also have a post- bronchodilator FEV$_1$/FVC<0.70 at screening.

These criteria ensure admission of GOLD 3 and 4 patients with exacerbation risk categories C and D (Global Strategy for the Diagnosis, Management and Prevention of COPD, Global Initiative for Chronic Obstructive Lung Disease (GOLD) 2013).

Individuals are not eligible to participate if they have a clinically important pulmonary disease other than COPD (e.g., active lung infection, clinically significant bronchiectasis, pulmonary fibrosis, cystic fibrosis, hypoventilation syndrome associated with obesity, lung cancer, alpha 1 anti-trypsin deficiency, or primary ciliary dyskinesia) or another diagnosed pulmonary or systemic disease that is associated with elevated peripheral eosinophil counts (e.g., allergic bronchopulmonary aspergillosis/mycosis, Churg-Strauss syndrome, or hypereosinophilic syndrome.) Individuals are also not eligible to participate if they have asthma as a primary or main diagnosis according to the Global Initiative for Asthma (GINA) guidelines or other accepted guidelines. However, individuals with a past medical history of asthma (e.g., childhood or adolescence) can be included. Individuals with unstable ischemic heart disease, arrhythmia, cardiomyopathy, heart failure, renal failure, uncontrolled hypertension, or any other relevant cardiovascular disorder are not eligible to participate. Individuals with lung volume reduction surgery within the 6 months prior to Visit 1 are not eligible to participate. Individuals using systemic corticosteroids, antibiotics, and/or hospitalization for a COPD exacerbation within 8 weeks prior to randomization or 4 weeks prior to enrollment (based on last dose of steroids or last date of hospitalization whatever occurred later) are not eligible to participate. Individuals receiving long term oxygen therapy (LTOT) with signs and/or symptoms of cor pulmonale, right ventricular failure or evidence by echocardiogram or pulmonary artery catheterization of moderate to severe pulmonary hypertension are not eligible to participate.

(b) DESIGN OF THE STUDIES

(i) TWO-DOSE STUDY

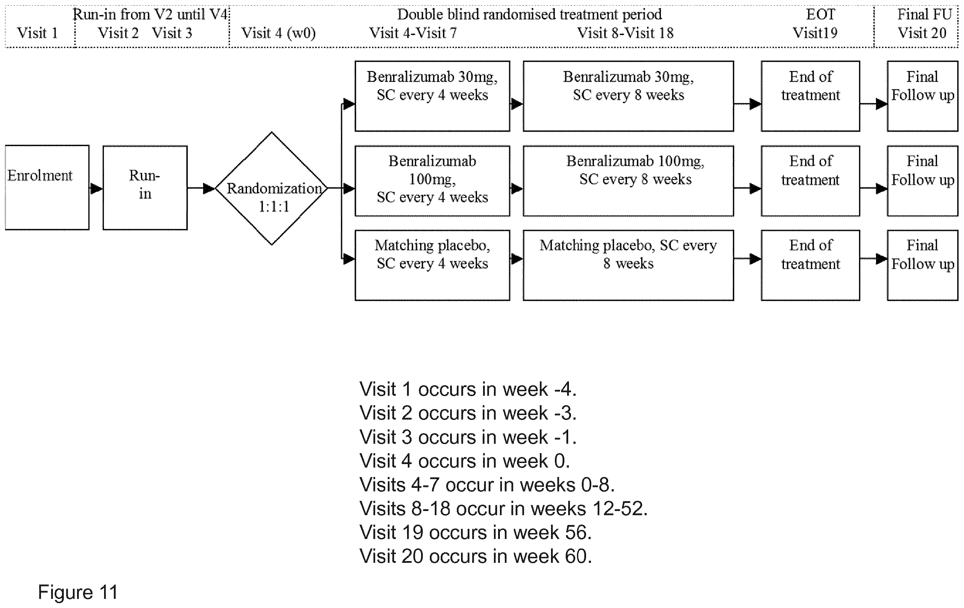

The two-dose study is a randomized, double-blind, placebo-controlled, parallel group, multicentre, phase III study in which multiple doses of benralizumab are administered subcutaneously to COPD patients. Benralizumab is administered at 30 mg and 100 mg doses every 4 weeks for the first 3 doses and then every 8 weeks thereafter. The study flow diagram is shown in Figure 11.

About 1743 subjects are recruited and stratified by country and blood eosinophil count (≥300/μL and <300/μL). The subjects are randomized into three treatment groups in a 1:1:1 ratio (benralizumab 30 mg: benralizumab 100 mg: placebo).

(ii) THREE-DOSE STUDY

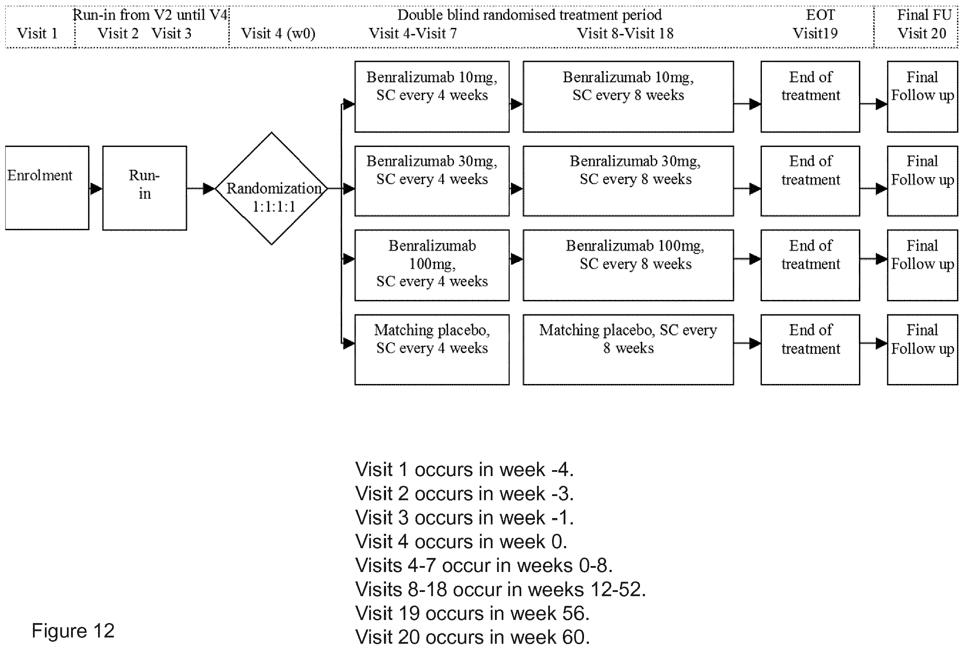

The three-dose study is a randomized, double-blind, double dummy, placebo-controlled, parallel group, multicentre, phase III study in which multiple doses of benralizumab are administered subcutaneously to COPD patients. Benralizumab is administered at 10 mg, 30 mg, and 100 mg doses every 4 weeks for the first 3 doses and then every 8 weeks thereafter. The study flow diagram is shown in Figure 12.

About 2324 subjects are recruited and stratified by country and blood eosinophil count ($\geq$300/$\mu$L and <300/$\mu$L). The subjects are randomized into four treatment groups in a 1:1:1:1 ratio (benralizumab 10 mg benralizumab 30 mg: benralizumab 100 mg: placebo).

(iii) TWO AND THREE-DOSE STUDIES

After the initial enrollment and confirmation of the entry criteria, subjects in the two-dose and three-dose studies enter a 1-week enrollment period and then proceed to the screening/run-in period for 3 weeks to allow adequate time for all of the eligibility criteria to be evaluated. During the run-in period, lung function is evaluated to determine if it meets the study eligibility criteria, and a laboratory test for absolute blood eosinophils is conducted (Visits 2 and 3).

In Visit 4, subjects who meet the eligibility criteria are randomized to a 56-week treatment period, and the first dose of the benralizumab or placebo is administered. Subjects have scheduled visits at 4-week intervals up to Visit 7 and then at 8-week intervals up to Visit 19. The last dose of benralizumab/placebo is administered at Week 48 (Visit 17). The end of treatment (EOT) visit occurs at Week 56. Subjects are maintained on their currently prescribed maintenance therapies from enrollment throughout the run-in and treatment period. Final follow-up visits are conducted at Week 60.

(c) SAFETY

Adverse events are monitored following administration of placebo or benralizumab. Other assessments included physical examination, vital sign monitoring, and laboratory measurements including hematology, chemistry, and urinalysis.

(d) EFFICACY

(i) COPD Exacerbations

In this study, a COPD exacerbation is defined as a worsening of symptoms that leads to any of the following:

- Use of systemic corticosteroids for at least 3 days (a single depot injectable dose of corticosteroids is considered equivalent to a 3-day course of systemic corticosteroids);
- Use of antibiotics; and/or
- An inpatient hospitalization due to COPD

The start of an exacerbation is defined as the start date of systemic corticosteroids or antibiotic treatment or hospital admission, whichever occurs earlier. The end date is defined as the last day of systemic corticosteroids or antibiotic treatment or hospital discharge, whichever occurs later. A COPD exacerbation that occurs $\leq$7 days of the last dose of systemic steroids (oral, IM, IV) or antibiotics will be counted as the same exacerbation event.

The annual exacerbation rate per subject is calculated, and standardized per 56-week period according to the following formula:

$$\text{Annual Exacerbation Rate} = \text{Number of Exacerbations} * 365.25 / (\text{Last follow-up date} - \text{Visit 4 Date} + 1).$$

The annual exacerbation rate in each of the two benralizumab dose groups is compared to annual exacerbation rate in the placebo group using a negative binomial model including covariates of treatment group, country, background group (ICS/LABA, LABA/LAMA, or ICS/LABA/LAMA), and the number of exacerbations in the year before the study. The logarithm of the follow up time is used as an offset variable in the model.

The time from randomization to the first COPD exacerbation is used as a supportive variable, and is calculated as follows:
Start Date of first COPD exacerbation - Date of Randomization +1.

The time to first COPD exacerbation for subjects who do not experience a COPD exacerbation during the treatment period will be censored at the date of their last visit for the 56-week double-blind treatment period, or at the time point after which an exacerbation could not be assessed (for lost-to-follow-up subjects).

This analysis is used to demonstrate that administration of benralizumab can reduce annual COPD exacerbation rates (e.g., in subjects with a baseline blood eosinophil count ≥300/μL).

(ii) Spirometry

Lung function ($FEV_1$ and FVC) is measured by spirometry. Subjects are instructed not to use their ICS/LABA, LABA, or LAMA medication within 12 hours or their rescue SABA medication (albuterol/salbutamol) within 6 hours of the scheduled spirometry.

Spirometry testing is initiated in the morning between 6:00 AM and 11:00 AM. All post-randomization spirometry assessments are performed within ± 1.5 hours of the time that the randomization spirometry was performed.

Post-BD spirometry is performed at Visit 2 for all subjects. Endpoint maximal bronchodilation is induced using albuterol (90 μg metered dose) or salbutamol (100 μg metered dose) with or without a spacer device up to a maximum of 4 inhalations within 30 minutes ± 15 minutes of the final pre-BD spirometry measurement. Post-BD spirometry is performed 20-30 minutes later. The subject's usual COPD morning maintenance therapy is not given until after the initial pre-medication, pre/post bronchodilator spirograms are complete.

The Global Lung Function Initiative (GLI) equations are used to determine the subjects predicted normal (PN) values. Quanjer et al., Multi ethnic reference values for spirometry for the 3-95 year age range: the global lung function 2012 equations, Report of the Global Lung Function Initiative (GLI), ERS Task Force to establish improved Lung Function Reference Values. (2012) doi: 10.1183/09031936.00080312.

$FEV_1$, expressed as percent of the PN value, is calculated as follows:

$$FEV_1\% \text{ of } PN = FEV_1 \text{ measured}/FEV_{1PN} \text{ x } 100.$$

The change from baseline to each of the post-randomization visits (post Visit 4) up to and including the end of 56-week double-blind treatment visit (Visit 19) is measured. The pre-bronchodilator measurement recorded at Visit 4 is used as baseline $FEV_1$. If the Visit 4 pre-bronchodilator measurement is missing, the last non-missing pre-bronchodilator value before Visit 4 is used as baseline instead.

A change from baseline in pre-dose/pre-bronchodilater $FEV_1$ at Week 56 (52 weeks after administration of the first dose of benralizumab or placebo) is compared between each of the two benralizumab dose regimen groups and placebo using a repeated measures analysis. Treatment groups are fitted as the explanatory variable. Country, background therapy (ICS/LABA, LABA/LAMA, or ICS/LABA/LAMA) and baseline pre- bronchodilater $FEV_1$ are fitted as covariates.

This analysis is used to demonstrate that administration of benralizumab can increase $FEV_1$ (e.g., in subjects with a baseline blood eosinophil count ≥300/μL).

(iii) Patient Reported Outcomes (PRO)

(1) St. George's Respiratory Questionnaire (SGRQ)

The SGRQ is a 50-item patient reported outcome (PRO) instrument developed to measure the health status of subjects with airway obstruction diseases (Jones et al., The St George's Respiratory Questionnaire. Respir Med. 85: Suppl B:25-31 (1991)). The questionnaire is divided into two parts: part 1 consists of 8 items pertaining to the severity of respiratory symptoms in the preceding 4 weeks; part 2 consists of 42 items related to the daily activity and psychosocial impacts of the individual's respiratory condition. The SGRQ yields a total score and three domain scores (symptoms, activity, and impacts). The total score indicates the impact of disease on overall health status. This total score is expressed as a percentage of overall impairment, in which 100 represents the worst possible health status and 0 indicates the best possible health status. Likewise, the domain scores range from 0 to 100, with higher scores indicative of greater impairment. Specific details on the scoring algorithms are provided in Jones et al., Eur Respir J 34: 648-654 (2009).

A change from baseline SGRQ at Week 56 (52 weeks after the first administration of benralizumab or placebo) is compared between each of the two benralizumab dose regimen groups and placebo using a repeated measures analysis. A responder is defined as an individual with a ≥4-point decrease (improvement) in SGRQ total score at Week 56.

This analysis is used to demonstrate that administration of benralizumab can improve (decrease) SGRQ scores (e.g., in subjects with a baseline blood eosinophil count ≥300/μL).

(2) Baseline/Transitional Dyspnea Index (BDI/TDI)

The BDI/TDI is an instrument developed to provide a multidimensional measure of dyspnea in relation to activities of daily living. Mahler et al., Chest 85:751-758 (1984). The Baseline Dyspnea Index (BDI) provides a measure of dyspnea at a single state, the baseline, and the Transitional Dyspnea Index (TDI) evaluates changes in dyspnea from the baseline state. The instrument consists of three components: functional impairment, magnitude of task, and magnitude of effort. For the BDI, each of these three components are rated in five grades from 0 (severe) to 4 (unimpaired), and are summed to form a baseline total score from 0 to 12. BDI is captured at baseline only. For the TDI, changes in dyspnea are rated for each component by seven grades from -3 (major deterioration) to +3 (major improvement), and are added to form a total TDI score from -9 to +9. Positive scores indicate an improvement, and a change from the BDI or a difference between treatments of 1 point has been estimated to constitute the minimum clinically important difference (MCID). Mahler et al., COPD 2: 99-103 (2005).

BDI and TDI scores are calculated to demonstrate that administration of benralizumab can improve respiratory symptoms (e.g., in subjects with a baseline blood eosinophil count ≥300/$\mu$L).

(3) COPD Assessment Test (CAT)

The CAT is an 8-item PRO developed to measure the impact of COPD on health status. Jones et al,. Eur Respir J 34: 648-654 (2009). The instrument uses semantic differential six-point response scales which are defined by contrasting adjectives to capture the impact of COPD. Content includes items related to cough, phlegm, chest tightness, breathlessness going up hills/stairs, activity limitation at home, confidence leaving home, sleep, and energy. A CAT total score is the sum of item responses. Scores range from 0-40 with higher scores indicative of greater COPD impact on health status.

CAT scores are calculated to demonstrate that administration of benralizumab can improve health-relate quality of life (e.g., in subjects with a baseline blood eosinophil count ≥300/$\mu$L).

(4) Exacerbations of Chronic Pulmonary Disease Tool - Patient-reported Outcome (EXACT-PRO) and EXACT-Respiratory Symptoms (E-RS)

The EXACT-PRO is a 14-item PRO instrument developed to assess the frequency, severity, and duration of COPD exacerbations. Jones et al., Chest 139:1388-1394 (2011); Leidy et al., Am J Respir Crit Care Med 183:323-329 (2011). The instrument was developed for daily, at home, administration using a handheld electronic device. Respondents are instructed to complete the diary each evening just prior to bedtime and to answer the questions while considering their experiences "today." The daily EXACT-PRO total score has a range of 0-100 with higher scores indicative of greater severity. Total score changes are used to identify the onset and recovery from an EXACT-PRO defined exacerbation event. In identifying event onset and recovery, the EXACT-PRO can provide information on event frequency and duration as well as event severity.

EXACT-PRO daily total scores as well as domain scores are calculated. The total score is used to identify event onset and recovery as well as the magnitude (severity) of the event. The baseline total score is the mean within subject score over the 7 days prior to randomization. A minimum of 4 days of data is required for calculating the baseline total score. To allow for improvement or deterioration in disease state over the course of the trial, the baseline total score is reset every 4 weeks in the absence of an EXACT-PRO defined event. Event frequency is calculated by comparing the baseline with daily total scores. Calculating event duration requires identification of the following five parameters: 1) onset; 2) three-day rolling average; 3) maximum observed value; 4) threshold for improvement; and 5) recovery. The severity of an event is indicated by the worst (highest) EXACT-PRO total score during an event.

EXACT-PRO scores are calculated to demonstrate that administration of benralizumab can decrease EXACT-PRO defined events (e.g., in subjects with a baseline blood eosinophil count ≥300/$\mu$L).

The E-RS is an 11-item PRO developed to evaluate the severity of respiratory symptoms of COPD (Sexton et al,. PRO evidence dossier to support the use of the E-RS to evaluate respiratory symptoms in patients with COPD. United BioSource Corporation; Bethesda, MD: May 2010; Sexton et al., Quantifying the severity of respiratory symptoms of COPD: reliability and validity of a patient diary. Poster presented at the American Thoracic Society International Meeting; May 2011: Denver, CO). The E-RS is a subset of items from the EXACT-PRO. The E-RS was designed to be captured as part of the daily EXACT-PRO assessment. Summation of E-RS item responses produces a total score ranging from 0 to 40, with higher scores indicating greater severity. In addition to the total score, symptom domain scores can be calculated for breathlessness (5 items; score range: 0-17), cough and sputum (3 items; score range: 0-11) and chest symptoms (3 items; score range: 0-11) by summing the responses of items within a respective domain. As with the total score, higher domain scores indicate greater severity.

Change from baseline in E-RS total score and domain scores at Week 56 are analyzed using a similar model as the model for change from baseline in pre-dose/pre-bronchodilator $FEV_1$. AUC of E-RS total score is analyzed by fitting an ANCOVA model with treatment, country, baseline value, and background therapy (ICS/LABA, LABA/LAMA, or ICS/LABA/LAMA) as covariates.

Individual daily E-RS total and subscale scores are calculated and summarized as a biweekly (14-day) mean. Data collected in the two-week period prior to randomization are used to calculate the individual E-RS total and subscale baseline means.

E-RS scores are calculated to demonstrate that administration of benralizumab can improve respiratory symptoms (e.g., in subjects with a baseline blood eosinophil count ≥300/μL).

Symptoms are assessed each morning for the purposes of a symptom worsening alert. Each morning subjects complete 3 questions pertaining to the major symptoms of a worsening event (dyspnea, sputum volume, and sputum color). Subjects reporting worsening of 1 or more of these symptoms triggers assessment of the minor symptoms of a worsening event (sore throat, cold, fever without other cause, cough, and wheeze). All questions will have a 24 hour recall period. Questions pertain to the severity of symptoms vs. their usual state and to the presence or absence of a symptom.

An alert is triggered if two or more major symptoms (dyspnea, sputum volume, and sputum color) worsen for two consecutive days or if one major symptom and one minor symptom (sore throat, cold, fever without other cause, cough, and wheeze) worsen for at least two consecutive days. When either of these criteria is met the subject is alerted to contact the study center as soon as possible for further evaluation. Likewise the study center will be alerted to contact the subject within approximately 24-48 hours if he or she has not yet contacted the center for further evaluation.

(5) Nocturnal Awakenings

Subjects report the occurrence of nocturnal awakenings due to COPD symptoms each morning. A single question with yes/no response options is used.

The number of nights with awakening due to COPD and requiring rescue medication is analyzed as the response variable by fitting an ANCOVA model to data. Treatment group is fitted as the explanatory variable, and country, baseline value and background therapy (ICS/LABA, LABA/LAMA, or ICS/LABA/LAMA) are fitted as covariates. This calculation is used to demonstrate that administration of benralizumab decreases awakening due to COPD (e.g., in subjects with a baseline blood eosinophil count ≥300/μL).

(6) Rescue Medication Use

Rescue medication usage including reliever inhaler and nebulizer use is captured twice daily. Inhaler usage is reported as the number of puffs in a given period, whereas nebulizer use is reported as the number of times. Rescue medication usage at night is assessed in the morning, and rescue medication used during the day is assessed in the evening.

Rescue medication use (average puffs/day) is analyzed using a similar model as described above for nocturnal awakenings. This analysis is used to demonstrate that administration of benralizumab decrease rescue mediation use (e.g., in subjects with a baseline blood eosinophil count ≥300/μL).

(7) Maintenance Medication Use

Maintenance medication adherence is assessed each evening via a single yes/no question. The subject is if they took their regularly scheduled inhaler (yes/no) and instructed not to consider instances of rescue inhaler usage when answering this question. This analysis is used to demonstrate that administration of benralizumab decreases maintenance mediation use (e.g., in subjects with a baseline blood eosinophil count ≥300/μL).

(8) Healthcare Resource Utilization

Broad-based health care utilization event information is collected.

For example, the annual rate of COPD exacerbations that are associated with an emergency room visit or a hospitalization is collected. In the statistical analysis, the number of COPD exacerbations that are associated with an emergency room visit or a hospitalization experienced by a subject during the 56-week double-blind treatment period is used as response variable, and the logarithm of the subject's corresponding follow-up time is used as an offset in the analysis to adjust for subjects having different exposure times during which the events occur. Maximum follow-up time is approximately 56 weeks. This analysis is used to demonstrate that administration of benralizumab decreases COPD exacerbations that are associated with emergency room visits or hospitilization (e.g., in subjects with a baseline blood eosinophil count ≥300/μL).

Those skilled in the art will recognize, or be able to ascertain using no more than routine experimentation, many equivalents to the specific aspects of the disclosure described herein. Such equivalents are intended to be encompassed by the following claims.

Various publications are cited herein, the disclosures of which are incorporated by reference in their entireties.

Although the foregoing invention has been described in some detail by way of illustration and example for purposes of clarity of understanding, it will be obvious that certain changes and modifications can be practiced within the scope of the appended claims.

SEQUENCE LISTING

SEQ ID NO:1

>US20100291073_1 Sequence 1 from Patent US 20100291073 Organism: Homo sapiens

DIQMTQSPSSLSASVGDRVTITCGTSEDIINYLNWYQQKPGKAPKLLIYHTSRLQSGVPSR

FSGSGSGTDFTLTISSLQP

EDFATYYCQQGYTLPYTFGQGTKVEIK


SEQ ID NO:2

>US20100291073_2 Sequence 2 from Patent US 20100291073 Organism: Homo sapiens

DIQMTQSPSSLSASVGDRVTITCGTSEDIINYLNWYQQKPGKAPKLLIYHTSRLQSGVPSR

FSGSGSGTDFTLTISSLQP

EDFATYYCQQGYTLPYTFGQGTKVEIKRTVAAPSVFIFPPSDEQLKSGTASVVCLLNNFY

PREAKVQWKVDNALQSGNSQ

ESVTEQDSKDSTYSLSSTLTLSKADYEKHKVYACEVTHQGLSSPVTKSFNRGEC


SEQ ID NO:3

>US20100291073_3 Sequence 3 from Patent US 20100291073 Organism: Homo sapiens

EVQLVQSGAEVKKPGASVKVSCKASGYTFTSYVIHWVRQRPGQGLAWMGYINPYNDG

TKYNERFKGKVTITSDRSTSTVY

MELSSLRSEDTAVYLCGREGIRYYGLLGDYWGQGTLVTVSS


SEQ ID NO:4

>US20100291073_4 Sequence 4 from Patent US 20100291073 Organism: Homo sapiens

EVQLVQSGAEVKKPGASVKVSCKASGYTFTSYVIHWVRQRPGQGLAWMGYINPYNDG

TKYNERFKGKVTITSDRSTSTVY

MELSSLRSEDTAVYLCGREGIRYYGLLGDYWGQGTLVTVSSASTKGPSVFPLAPSSKSTS

GGTAALGCLVKDYFPEPVTV

SWNSGALTSGVHTFPAVLQSSGLYSLSSVVTVPSSSLGTQTYICNVNHKPSNTKVDKKVE

PKSCDKTHTCPPCPAPELLG

GPSVFLFPPKPKDTLMISRTPEVTCVVVDVSHEDPEVKFNWYVDGVEVHNAKTKPREEQ

YNSTYRVVSVLTVLHQDWLNG

KEYKCKVSNKALPAPIEKTISKAKGQPREPQVYTLPPSRDELTKNQVSLTCLVKGFYPSDI

AVEWESNGQPENNYKTTPP

VLDSDGSFFLYSKLTVDKSRWQQGNVFSCSVMHEALHNHYTQKSLSLSPGK


SEQ ID NO:5

>US20100291073_5 Sequence 5 from Patent US 20100291073 Organism: Homo sapiens

DLLPDEKISLLPPVNFTIKVTGLAQVLLQWKPNPDQEQRNVNLEYQVKINAPKEDDYET

RITESKCVTILHKGFSASVRT

ILQNDHSLLASSWASAELHAPPGSPGTSIVNLTCTTNTTEDNYSRLRSYQVSLHCTWLVG

TDAPEDTQYFLYYRYGSWTE

ECQEYSKDTLGRNIACWFPRTFILSKGRDWLAVLVNGSSKHSAIRPFDQLFALHAIDQINP

PLNVTAEIEGTRLSIQWEK

PVSAFPIHCFDYEVKIHNTRNGYLQIEKLMTNAFISIIDDLSKYDVQVRAAVSSMCREAGL

WSEWSQPIYVGNDEHKPLR

EWFVIVIMATICFILLILSLICKICHLWIKLFPPIPAPKSNIKDLFVTTNYEKAGSSETEIEVIC

YIEKPGVETLEDSVF


SEQ ID NO:6

>US20100291073_6 Sequence 6 from Patent US 20100291073 Organism: Mus musculus

DLLNHKKFLLLPPVNFTIKATGLAQVLLHWDPNPDQEQRHVDLEYHVKINAPQEDEYDT

RKTESKCVTPLHEGFAASVRT

ILKSSHTTLASSWVSAELKAPPGSPGTSVTNLTCTTHTVVSSHTHLRPYQVSLRCTWLVG

KDAPEDTQYFLYYRFGVLTE

KCQEYSRDALNRNTACWFPRTFINSKGFEQLAVHINGSSKRAAIKPFDQLFSPLAIDQVN

PPRNVTVEIESNSLYIQWEK

PLSAFPDHCFNYELKIYNTKNGHIQKEKLIANKFISKIDDVSTYSIQVRAAVSSPCRMPGR

WGEWSQPIYVGKERKSLVE

WHLIVLPTAACFVLLIFSLICRVCHLWTRLFPPVPAPKSNIKDLPVVTEYEKPSNETKIEVV

HCVEEVGFEVMGNSTF

SEQ ID NO:7  - VH CDR1

SYVIH

SEQ ID NO:8 – VH CDR2

YINPYNDGTKYNERFKG

SEQ ID NO:9 – VH CDR3

EGIRYYGLLGDY

SEQ ID NO:10 – VL CDR1

GTSEDIINYLN

SEQ ID NO:11 – VL CDR2

HTSRLQS

SEQ ID NO:12 – VL CDR3

QQGYTLPYT

EMBODIMENTS:

1. A method of treating chronic obstructive pulmonary disease (COPD) in a human COPD patient, comprising administering to the patient a dose of 100 mg of benralizumab or an antigen-binding fragment thereof.

2. A method of reducing the exacerbation rate of COPD comprising administering to a human COPD patient an effective amount of benralizumab or an antigen-binding fragment thereof, wherein the patient has a blood eosinophil count of at least 200 eosinophils/$\mu$L prior to the administration.

3. A method of reducing the exacerbation rate of COPD comprising administering to a human COPD patient an effective amount of benralizumab or an antigen-binding fragment thereof, wherein the patient has severe or very severe COPD as defined by the Global Initiative for Chronic Obstructive Lung Disease (GOLD).

4. A method of increasing forced expiratory volume in one second ($FEV_1$) in a human COPD patient comprising administering an effective amount of benralizumab or an antigen-binding fragment thereof to the patient.

5. A method of increasing forced vital capacity (FVC) in a human COPD patient comprising administering an effective amount of benralizumab or an antigen-binding fragment thereof to the patient.

6. A method of improving a COPD questionnaire score assessing COPD symptoms in a human COPD patient comprising administering an effective amount of benralizumab or an antigen-binding fragment thereof to the patient.

7. The method of any one of embodiments 2-6, wherein the benralizumab or antigen-binding fragment is administered in a dose of 100 mg.

8. The method of any one of embodiments 1 and 3-7, wherein the patient has a blood eosinophil count of at least 200 eosinophils/$\mu$L prior to the administration.

9. The method of embodiment 2 or 8, wherein the patient has a blood eosinophil count of at least 300 eosinophils/$\mu$L prior to the administration.

10. The method of embodiment 2 or 9, wherein the patient has a blood eosinophil count of at least 400 eosinophils/$\mu$L prior to the administration.

11. The method of any one of embodiments 1, 2, and 4-10, wherein the patient has severe or very severe COPD as defined by GOLD.

12. The method of embodiment 3 or 11, wherein the patient has very severe COPD as defined by GOLD.

13. The method of any one of embodiments 1 and 4-12, wherein the administration reduces the exacerbation rate of COPD.

14. The method of any one of embodiments 2, 3, 7, 9, 11, 12, and 13, wherein the exacerbation rate is reduced by at least 30%.

15. The method of embodiment 14, wherein the exacerbation rate is reduced by about 34%

16. The method of embodiment 14, wherein the exacerbation rate is reduced by at least 40%.

17. The method of embodiment 16, wherein the exacerbation rate is reduced by about 47%.

18. The method of embodiment 16, wherein the exacerbation rate is reduced by at least 50%.

19. The method of embodiment 18, wherein the exacerbation rate is reduced by about 57%.

20. The method of any one of embodiments 2, 3, 7-19, wherein the exacerbation rate is reduced within a year from the first administration of the benralizumab or antigen-binding fragment thereof.

21. The method of any one of embodiments 1-3 and 5-20, wherein the administration increases the patient's $FEV_1$.

22. The method of embodiment 4 or 21, wherein the increased $FEV_1$ is a pre-bronchodilator $FEV_1$.

23. The method of embodiment 22, wherein the pre-bronchodilator $FEV_1$ is increased by at least 10%.

24. The method of embodiment 23, wherein the pre-bronchodilator $FEV_1$ is increased by about 12%.

25. The method of embodiment 4 or 21, wherein the increased $FEV_1$ is a post-bronchodilator $FEV_1$.

26. The method of embodiment 25, wherein the post-bronchodilator $FEV_1$ is increased by at least 5%.

27. The method of embodiment 26, wherein the post-bronchodilator $FEV_1$ is increased by about 7%.

28. The method of any one of embodiments 4 and 21-26, wherein the pre-bronchodilator $FEV_1$ and the post-bronchodilator $FEV_1$ increase.

29. The method of any one of embodiments 4 and 21-28, wherein the $FEV_1$ is increased within a year from the first administration of the benralizumab or antigen-binding fragment thereof.

30. The method of any one of embodiments 1-4 and 6-29, wherein the administration increases the patient's FVC.

31. The method of embodiment 5 or 30, wherein the increased FVC is a pre-bronchodilator FVC.

32. The method of embodiment 5 or 30, wherein the increased FVC is a post-bronchodilator FVC.

33. The method of any one of embodiments 5 and 30-32, wherein the pre-bronchodilator FVC and the post-bronchodilator FVC increase.

34. The method of any one of embodiments 5 and 30-33, wherein the FVC is increased by at least 3%.

35. The method of any one of embodiments 5 and 30-34, wherein the FVC is increased within a year from the first administration of the benralizumab or antigen-binding fragment thereof.

36. The method of any one of embodiments 1-5 and 7-35, wherein the administration improves a COPD questionnaire score assessing COPD symptoms.

37. The method of embodiment 6 or 36, wherein the COPD questionnaire is the COPD-Specific Saint George's Respiratory Questionnaire (SGRQ-C).

38. The method of embodiment 6, 36, or 37, wherein the patient's SGRQ-C (symptom) score decreases by at least 9.

39. The method of any one of embodiments 5 and 36-38, wherein the COPD questionnaire score assessing COPD symptoms improve with a year from the first administration of the benralizumab or antigen-binding fragment thereof.

40. The method of any one of embodiments 1-39, wherein the patient has a history of exacerbations.

41. The method of embodiment 40, wherein the history of exacerbations comprises at least one exacerbation in the year prior to the administration of the benralizumab or antigen-binding fragment thereof.

42. The method of any one of embodiments 1-41, wherein the patient had an $FEV_1$ < 80% predicted prior to the administration of the benralizumab or antigen-binding fragment thereof.

43. The method of any one of embodiments 1-42, wherein the patient had an $FEV_1$/ forced vital capacity (FVC) < 0.70 prior to the administration of the benralizumab or antigen-binding fragment thereof.

44. The method of any one of embodiments 1-43, wherein the patient uses corticosteroids, long-acting $\beta$2 agonists, and tiotropium.

45. The method of any one of embodiments 1-44, wherein at least two doses of the benralizumab or antigen-binding fragment thereof are administered.

46. The method of embodiment 45, wherein the first dose of benralizumab or antigen-binding fragment thereof is administered at day zero and the second dose is administered at 4 weeks.

47. The method of embodiment 45, wherein at least one dose of the benralizumab or antigen-binding fragment thereof is administered at an interval of 8 weeks after the previous dose.

48. The method of any one of embodiments 45-47, wherein the benralizumab or antigen-binding fragment thereof is administered with at least one four-week dosing interval and then with at least one eight-week dosing interval.

49. The method of embodiment 48, wherein the benralizumab or antigen-binding fragment thereof is administered with three four-week dosing intervals and then at eight-week dosing intervals.

50. The method of any one of embodiments 1-49, wherein the administration is subcutaneous.

51. A method of treating COPD in a human COPD patient, comprising administering to the patient a dose of 100 mg of benralizumab or an antigen-binding fragment thereof, wherein the patient has a blood eosinophil count of at least 200 eosinophils/$\mu$L prior to the administration.

52. The method of embodiment 51, wherein the patient has a blood eosinophil count of at least 300 eosinophils/$\mu$L prior to the administration.

53. The method of embodiment 52, wherein the patient has a blood eosinophil count of at least 400 eosinophils/$\mu$L prior to the administration.

54. The method of any one of embodiments 51-53, wherein the benralizumab or antigen-binding fragment thereof is administered with at least one four-week dosing interval and then with at least one eight-week dosing interval.

55. The method of any one of embodiments 51-54, wherein the administration of the benralizumab or antigen-binding fragment thereof decreases the exacerbation rate of chronic obstructive pulmonary disease (COPD), increases the patient's $FEV_1$, improves a COPD questionnaire score assessing COPD symptoms, or a combination thereof.

56. The method of embodiment 55, wherein the administration of the benralizumab or antigen-binding fragment thereof decreases the exacerbation rate of chronic obstructive pulmonary disease (COPD), increases the patient's $FEV_1$, and improves a COPD questionnaire score assessing COPD symptoms.

57. A method of reducing the exacerbation rate of COPD comprising administering to a human COPD patient an effective amount of benralizumab or an antigen-binding fragment thereof, wherein the patient has a blood eosinophil count of at least 200 eosinophils/$\mu$L prior to the administration and wherein the exacerbation rate is reduced by at least 30%.

58. The method of embodiment 57, wherein the exacerbation rate is reduced by about 34%.

59. A method of reducing the exacerbation rate of COPD comprising administering to a human COPD patient an effective amount of benralizumab or an antigen-binding fragment thereof, wherein the patient has a blood eosinophil count of at least 300 eosinophils/$\mu$L prior to the administration and wherein the exacerbation rate is reduced by at least 50%.

60. The method of embodiment 59, wherein the exacerbation rate is reduced by about 57%

61. A method of reducing the exacerbation rate of COPD comprising administering to a human COPD patient an effective amount of benralizumab or an antigen-binding fragment thereof, wherein the patient has severe or very severe COPD as defined by GOLD and wherein the exacerbation rate is reduced by at least 40%.

62. The method of embodiment 61, wherein the exacerbation rate is reduced by about 47%

63. A method of increasing $FEV_1$ in a human COPD patient comprising administering an effective amount of benralizumab or an antigen-binding fragment thereof to the patient, wherein the patient has a blood eosinophil count of at least 200 eosinophils/$\mu$L prior to the administration.

64. A method of increasing $FEV_1$ in a human COPD patient comprising administering an effective amount of benralizumab or an antigen-binding fragment thereof to the patient, wherein the patient uses corticosteroids, long-acting $\beta2$ agonists, and tiotropium.

65. The method of embodiment 63 or 64, wherein the pre-bronchodilator $FEV_1$ increases.

66. The method of embodiment 65, wherein the pre-bronchodilator $FEV_1$ increases by at least 15%.

67. The method of embodiment 63 or 64, wherein the post-bronchodilator $FEV_1$ increases.

68. The method of embodiment 67, wherein the post-bronchodilator $FEV_1$ increases by at least 10%.

69. The method of any one of embodiments 63-68, wherein the pre-bronchodilator $FEV_1$ increases and the post-bronchodilator $FEV_1$ increases.

70. The method of embodiment 69, wherein the pre-bronchodilator $FEV_1$ increases by at least 15% and the post-bronchodilator $FEV_1$ increases by at least 10%.

71. A method of increasing $FEV_1$ in a human COPD patient comprising administering an effective amount of benralizumab or an antigen-binding fragment thereof to the patient, wherein the patient has severe or very severe COPD as defined by GOLD.

72. The method of embodiment 71, wherein the pre-bronchodilator $FEV_1$ increases.

73. The method of embodiment 72, wherein the pre-bronchodilator $FEV_1$ increases by at least 20%.

74. The method of embodiment 71, wherein the post-bronchodilator $FEV_1$ increases.

75. The method of embodiment 74, wherein the post-bronchodilator $FEV_1$ increases by at least 15%.

76. The method of any one of embodiments 71-75, wherein the pre-bronchodilator $FEV_1$ increases and the post-bronchodilator $FEV_1$ increases.

77. The method of embodiment 76, wherein the pre-bronchodilator $FEV_1$ increases by at least 20% and the post-bronchodilator $FEV_1$ increases by at least 15%.

78. The method of any one of embodiments 2-6, 8-50, or 57-77 wherein the benralizumab or antigen-binding fragment thereof is administered in a dose of 30 mg.

79. The method of any one of embodiments 2-6, 8-50, or 57-77, wherein the benralizumab or antigen-binding fragment thereof is administered in a dose of 10 mg.

80. The method of any one of embodiments 1-79, wherein the administration of benralizumab or the antigen-binding fragment thereof reduces the annual COPD exacerbation rate.

81. The method of any one of embodiments 1-80, wherein the administration of benralizumab or the antigen-binding fragment thereof improves a Specific Saint George's Respiratory Questionnaire (SGRQ) score.

82. A method of treating COPD in a human COPD patient, comprising administering to the patient a dose of 30 mg of benralizumab or an antigen-binding fragment thereof.

83. A method of treating chronic obstructive pulmonary disease (COPD) in a human COPD patient, comprising administering to the patient a dose of 10 mg of benralizumab or an antigen-binding fragment thereof.

84. The method of embodiment 82 or 83, wherein the administration of benralizumab or the antigen-binding fragment thereof reduces the annual COPD exacerbation rate.

85. A method of reducing the annual COPD exacerbation rate, comprising administering to a human COPD patient an effective amount of benralizumab or an antigen-binding fragment thereof.

86. The method of any one of embodiments 82-85, wherein the patient has a blood eosinophil count of at least 200 eosinophils/$\mu$L prior to the administration.

87. The method of any one of embodiments 82-85, wherein the patient has a blood eosinophil count of at least 300 eosinophils/$\mu$L prior to the administration.

88. The method of any one of embodiments 1, 3-7, 11-50, 61, 63, or 64-85, wherein the patient has a blood eosinophil count of less than 150 eosinophils/$\mu$L prior to the administration.

89. The method of any one of embodiments 1-8, 11-51, 53-57, or 61-85, wherein the patient has a blood eosinophil count of less than 300 eosinophils/$\mu$L prior to the administration.

90. The method of any one of embodiments 1, 3-7, 11-50, 61, 62, or 64-85, wherein the patient has a blood eosinophil count of 150-300 eosinophils/$\mu$L prior to the administration.

91. The method of any one of embodiments 1-85, wherein the patient has a blood eosinophil count of 300-450 eosinophils/$\mu$L prior to the administration.

92. The method of any one of embodiments 1-85, wherein the patient has a blood eosinophil count of at least 450 eosinophils/$\mu$L prior to the administration.

93. The method of any one of embodiments 1-92, wherein the patient uses an inhaled corticosteroids (ICS) and a long-acting beta agonist (LABA).

94. The method of any one of embodiments 1-92, wherein the patient uses a LABA and long-acting muscarinic antagonist (LAMA).

95. The method of any one of embodiments 1-92, wherein the patient uses ICS/LABA/LAMA.

96. The method of any one of embodiments 82-95, wherein the patient has an $FEV_1 < 50\%$ of the predicted normal value prior to the administration.

97. The method of any one of embodiments 82-96, wherein the patient has a history of at least 1 COPD exacerbation in the year prior to the administration.

98. The method of any one of embodiments 82-97, wherein the patient has severe or very severe COPD as defined by GOLD.

99. The method of any one of embodiments 82-98, wherein at least two doses of the benralizumab or antigen-binding fragment thereof are administered.

100. The method of embodiment 99, wherein the first dose of benralizumab or antigen-binding fragment thereof is administered at day zero and the second dose is administered at 4 weeks.

101. The method of embodiment 100, wherein at least one dose of the benralizumab or antigen-binding fragment thereof is administered at an interval of 8 weeks after the previous dose.

102. The method of any one of embodiments 99-101, wherein the benralizumab or antigen-binding fragment thereof is administered with at least one four-week dosing interval and then with at least one eight-week dosing interval.

103. The method of embodiment 102, wherein the benralizumab or antigen-binding fragment thereof is administered with three four-week dosing intervals and then at eight-week dosing intervals.

104. The method of any one of embodiments 1-103, wherein the administration is subcutaneous.

**Claims**

1. Benralizumab for use in a method of treating chronic obstructive pulmonary disease (COPD) in a human COPD patient, wherein a dose of 10 mg, 30 mg, or 100 mg of benralizumab or an antigen-binding fragment thereof is administered to the patient.

2. Benralizumab for use of claim 1, wherein the patient has a blood eosinophil count of at least 200 eosinophils/$\mu$L prior to the administration.

3. Benralizumab for use of claim 2, wherein the patient has a blood eosinophil count of at least 300 eosinophils/$\mu$L prior to the administration.

4. Benralizumab for use of claim 3, wherein the patient has a blood eosinophil count of at least 400 eosinophils/$\mu$L prior to the administration.

5. Benralizumab for use of any one of claims 1-4, wherein the patient has severe or very severe COPD as defined by GOLD.

6. Benralizumab for use of any one of claims 1-5, wherein the administration reduces the exacerbation rate of COPD.

7. Benralizumab for use of any one of claims 1-6, wherein the administration increases the patient's FEV1.

8. Benralizumab for use of any one of claims 1-7, wherein the administration increases the patient's FVC.

9. Benralizumab for use of any one of claims 1-8, wherein the administration improves a COPD questionnaire score assessing COPD symptoms.

10. Benralizumab for use of any one of claims 1-9, wherein the patient uses corticosteroids, long-acting $\beta$2 agonists, and tiotropium.

11. Benralizumab for use of any one of claims 1-10, wherein the benralizumab or antigen-binding fragment thereof is administered with three four-week dosing intervals and then at eight-week dosing intervals.

12. Benralizumab for use of any one of claims 1-11, wherein the administration is subcutaneous.

Figure 1

| | Placebo N / Rate | Benralizumab N / Rate | TV | Rate Reduction [95% CI] |
|---|---|---|---|---|
| Overall (N=101) | 50 / 0.91 | 51 / 0.93 | | −2% [−51%, 31%] |
| Peripheral Blood Eos | | | | |
| >= 200 (N=46) | 24 / 0.97 | 22 / 0.63 | | 35% [−24%, 65%] |
| < 200 (N=53) | 25 / 0.78 | 28 / 1.19 | | −53% [−162%, 11%] |
| >= 300 (N=25) | 8 / 0.93 | 17 / 0.33 | | 65% [−2%, 88%] |
| < 300 (N=74) | 41 / 0.86 | 33 / 1.27 | | −47% [−127%, 4%] |
| 200 − 300 (N=21) | 16 / 0.99 | 5 / 1.67 | | −69% [−280%, 24%] |

Rate Reduction (%)

Figure 2

Figure 3

EP 3 485 902 A1

| | Placebo N/Estimate | Benralizumab N/Estimate | Difference [95%CI] |
|---|---|---|---|
| Overall | 46/-0.046 | 42/0.091 | 0.137[0.010,0.264] |
| Overall w/o outlier | 46/-0.046 | 41/0.055 | 0.101[0.004,0.198] |
| EOS>=200 | 21/-0.045 | 19/0.203 | 0.249[0.009,0.489] |
| EOS>=200 w/o outlier | 21/-0.045 | 18/0.122 | 0.167[0.011,0.324] |
| EOS<200 | 24/-0.029 | 23/-0.000 | 0.029[-0.099,0.157] |
| EOS>=300 | 8/-0.204 | 14/0.204 | 0.283[-0.158,0.723] |
| EOS>=300 w/o outlier | 13/0.097 | 13/0.097 | 0.173[-0.084,0.429] |
| EOS<300 | 37/-0.029 | 28/0.034 | 0.063[-0.041,0.166] |
| EOS 200-300 | 13/-0.017 | 5/0.148 | 0.165[-0.042,0.371] |

Treatment Difference (L): -0.2 0.0 0.2 0.4 0.6 0.8

Figure 4

Figure 5

Figure 6

Figure 7

EP 3 485 902 A1

[1] DATA BEYOND DAY 337 (MARKED BY THE VERTICAL REFERENCE LINE) OCCUR AFTER THE LAST SCHEDULED DOSE OF STUDY DRUG. BSL-BASELINE, DX-DAY X.

Figure 8

[1] DATA BEYOND DAY 337 (MARKED BY THE VERTICAL REFERENCE LINE) OCCUR AFTER THE LAST SCHEDULED DOSE OF STUDY DRUG. BSL-BASELINE, DX-DAY X.

Figure 9

Figure 10

Figure 11

| Run-in from V2 until V4 | | Double blind randomised treatment period | | EOT | Final FU |
|---|---|---|---|---|---|
| Visit 1 | Visit 2  Visit 3 | Visit 4 (w0) | Visit 4-Visit 7 | Visit 8-Visit 18 | Visit19 | Visit 20 |

Enrolment → Run-in → Randomization 1:1:1:1 →

- Benralizumab 10mg, SC every 4 weeks → Benralizumab 10mg, SC every 8 weeks → End of treatment → Final Follow up
- Benralizumab 30mg, SC every 4 weeks → Benralizumab 30mg, SC every 8 weeks → End of treatment → Final Follow up
- Benralizumab 100mg, SC every 4 weeks → Benralizumab 100mg, SC every 8 weeks → End of treatment → Final Follow up
- Matching placebo, SC every 4 weeks → Matching placebo, SC every 8 weeks → End of treatment → Final Follow up

Visit 1 occurs in week -4.
Visit 2 occurs in week -3.
Visit 3 occurs in week -1.
Visit 4 occurs in week 0.
Visits 4-7 occur in weeks 0-8.
Visits 8-18 occur in weeks 12-52.
Visit 19 occurs in week 56.
Visit 20 occurs in week 60.

Figure 12

Europäisches
Patentamt

European
Patent Office

Office européen
des brevets

# EUROPEAN SEARCH REPORT

Application Number

EP 18 18 9626

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/143878 A1 (MEDIMMUNE LLC [US]; BIOWA INC [US]; KOIKE MASAMICHI [US]; SPITALNY GEO) 27 November 2008 (2008-11-27) | 1-4,6-9 | INV. A61K39/00 A61K39/395 G01N33/48 A61P11/00 |
| Y | * the whole document * * in particular, page 32 * | 5,10-12 | |
| X | WO 2012/158954 A1 (MEDIMMUNE LLC [US]; GOSSAGE DAVID [US]; KHATRY DEEPAK B [US]; GEBA GRE) 22 November 2012 (2012-11-22) | 1,6-9 | |
| Y | * the whole document * * in particular, pages 3, 8, 9 and 68 * | 5,10-12 | |
| Y | WO 2012/049278 A1 (MEDIMMUNE LTD [GB]; FAGGIONI RAFFAELLA [US]; MAY RICHARD [GB]; KELL CH) 19 April 2012 (2012-04-19) * the whole document * * in particular, page 17-18 * | 5,10-12 | |
| A | JONES PAUL W ET AL: "Characteristics of a COPD population categorised using the GOLD framework by health status and exacerbations", RESPIRATORY MEDICINE, BAILLIERE TINDALL, LONDON, GB, vol. 108, no. 1, 30 August 2013 (2013-08-30), pages 129-135, XP028816091, ISSN: 0954-6111, DOI: 10.1016/J.RMED.2013.08.015 * the whole document * | 5 | TECHNICAL FIELDS SEARCHED (IPC) C07K A61P |

-/--

The present search report has been drawn up for all claims

| Place of search | Date of completion of the search | Examiner |
|---|---|---|
| Munich | 11 February 2019 | Pérez-Mato, Isabel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | N. A. MOLFINO ET AL: "Molecular and clinical rationale for therapeutic targeting of interleukin-5 and its receptor", CLINICAL & EXPERIMENTAL ALLERGY, vol. 42, no. 5, 1 May 2012 (2012-05-01), pages 712-737, XP055026155, ISSN: 0954-7894, DOI: 10.1111/j.1365-2222.2011.03854.x * the whole document * * in particular, pages 724-725 and 728 * | 1-12 | |

TECHNICAL FIELDS
SEARCHED      (IPC)

The present search report has been drawn up for all claims

| Place of search | Date of completion of the search | Examiner |
|---|---|---|
| Munich | 11 February 2019 | Pérez-Mato, Isabel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 9626

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2019

| Patent document cited in search report | Publication date | Patent family member(s) | | Publication date |
|---|---|---|---|---|
| WO 2008143878 A1 | 27-11-2008 | AU | 2008255027 A1 | 27-11-2008 |
| | | AU | 2013273774 A1 | 16-01-2014 |
| | | AU | 2016273830 A1 | 05-01-2017 |
| | | BR | PI0811526 A2 | 16-05-2017 |
| | | CA | 2685222 A1 | 27-11-2008 |
| | | CA | 2986531 A1 | 27-11-2008 |
| | | CN | 101848732 A | 29-09-2010 |
| | | CN | 103223167 A | 31-07-2013 |
| | | CY | 1117594 T1 | 26-04-2017 |
| | | DK | 2068927 T3 | 18-01-2016 |
| | | DK | 3072525 T3 | 30-04-2018 |
| | | EP | 2068927 A1 | 17-06-2009 |
| | | EP | 3072525 A1 | 28-09-2016 |
| | | ES | 2558689 T3 | 08-02-2016 |
| | | ES | 2666165 T3 | 03-05-2018 |
| | | HK | 1132658 A1 | 28-10-2016 |
| | | HK | 1148220 A1 | 14-03-2014 |
| | | HK | 1187542 A1 | 06-05-2016 |
| | | HR | P20160058 T1 | 22-04-2016 |
| | | HR | P20180611 T1 | 01-06-2018 |
| | | HU | E026728 T2 | 28-06-2016 |
| | | HU | E036885 T2 | 28-08-2018 |
| | | IL | 202102 A | 31-08-2015 |
| | | IL | 240179 A | 31-12-2018 |
| | | JP | 2010527356 A | 12-08-2010 |
| | | JP | 2016034940 A | 17-03-2016 |
| | | JP | 2018021017 A | 08-02-2018 |
| | | KR | 20100022468 A | 02-03-2010 |
| | | KR | 20150107890 A | 23-09-2015 |
| | | KR | 20160010891 A | 28-01-2016 |
| | | LT | 3072525 T | 10-05-2018 |
| | | NZ | 599278 A | 20-12-2013 |
| | | PL | 3072525 T3 | 31-07-2018 |
| | | PT | 2068927 E | 10-02-2016 |
| | | PT | 3072525 T | 20-04-2018 |
| | | RU | 2009146125 A | 20-06-2011 |
| | | RU | 2014105496 A | 20-08-2015 |
| | | SG | 10201503254T A | 29-06-2015 |
| | | SI | 3072525 T1 | 29-06-2018 |
| | | US | 2010291073 A1 | 18-11-2010 |
| | | US | 2012148575 A1 | 14-06-2012 |
| | | US | 2014004109 A1 | 02-01-2014 |
| | | WO | 2008143878 A1 | 27-11-2008 |
| WO 2012158954 A1 | 22-11-2012 | EP | 2710370 A1 | 26-03-2014 |
| | | US | 2012328606 A1 | 27-12-2012 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                   EP 18 18 9626

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2019

| Patent document cited in search report | Publication date | Patent family member(s) | Publication date |
|---|---|---|---|
| | | WO    2012158954 A1 | 22-11-2012 |
| WO 2012049278    A1 | 19-04-2012 | AU    2011315499 A1 | 02-05-2013 |
| | | BR  112013009055 A2 | 19-07-2016 |
| | | CA    2814431 A1 | 19-04-2012 |
| | | CN    103328506 A | 25-09-2013 |
| | | DK    2627673 T3 | 06-11-2017 |
| | | EP    2627673 A1 | 21-08-2013 |
| | | ES    2645368 T3 | 05-12-2017 |
| | | HU    E034005 T2 | 29-01-2018 |
| | | JP    2013544235 A | 12-12-2013 |
| | | RU    2013122123 A | 20-11-2014 |
| | | US    2013281876 A1 | 24-10-2013 |
| | | WO    2012049278 A1 | 19-04-2012 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100291073 A1
- US 6018032 A

- US 20100291073 A

### Non-patent literature cited in the description

- **ANTHONISEN et al.** *Ann. Int. Med.,* 1987, vol. 106, 196-204
- **AARON et al.** *Chest,* 2002, vol. 121, 688-96
- **JONES et al.** *Respir. Med.,* 1991, vol. 85
- **MEGURO et al.** *Chest,* 2007, vol. 132, 456-63
- **GUYATT et al.** *Thorax,* 1987, vol. 42, 773-8
- **WILLIAMS et al.** *Thorax,* 2001, vol. 56, 954-9
- **ANTHONISEN et al.** *Ann. Int. Med,* 1987, vol. 106, 196-204
- **QUANJER et al.** Multi ethnic reference values for spirometry for the 3-95 year age range: the global lung function 2012 equations, Report of the Global Lung Function Initiative (GLI), ERS Task Force to establish improved Lung. *Function Reference Values.,* 2012
- **JONES et al.** The St George's Respiratory Questionnaire. *Respir Med.,* 1991, vol. 85, 25-31

- **JONES et al.** *Eur Respir J,* 2009, vol. 34, 648-654
- **MAHLER et al.** *Chest,* 1984, vol. 85, 751-758
- **MAHLER et al.** *COPD,* 2005, vol. 2, 99-103
- **JONES et al.** *Chest,* 2011, vol. 139, 1388-1394
- **LEIDY et al.** *Am J Respir Crit Care Med,* 2011, vol. 183, 323-329
- **SEXTON et al.** PRO evidence dossier to support the use of the E-RS to evaluate respiratory symptoms in patients with COPD. United BioSource Corporation, May 2010
- **SEXTON et al.** Quantifying the severity of respiratory symptoms of COPD: reliability and validity of a patient diary. Poster presented at the American Thoracic Society International Meeting, May 2011